# EUROPÄISCHE PATENTSCHRIFT

(11) **EP 2 067 811 B1**
(45) Veröffentlichungstag und Bekanntmachung des Hinweises auf die Patenterteilung: **26.09.2018**
(21) Anmeldenummer: 08165900.5
(22) Anmeldetag: 06.10.2008
(51) Int. Cl.: C08G 77/38, C08L 83/10, C09D 183/10, C08F 283/12, C08L 51/08, C09D 151/08

(54) **SILICON(METH-)ACRYLAT-PARTIKEL, VERFAHREN ZU DEREN HERSTELLUNG SOWIE DEREN VERWENDUNG**
SILICON (METH-)ACRYLATE PARTICLES, METHOD FOR THEIR MANUFACTURE AND APPLICATION THEREOF
PARTICULES DE SILICONE (MÉTH-)ACRYLATE, SON PROCÉDÉ DE FABRICATION ET D'UTILISATION

(30) Priorität: 06.12.2007 DE 102007058713
(43) Veröffentlichungstag der Anmeldung: 10.06.2009
(73) Patentinhaber: Evonik Degussa GmbH, 45128 Essen (DE)
(72) Erfinder: Venzmer, Joachim, 45239 Essen (DE); Meyer, Jürgen, 45134 Essen (DE); Naumann, Matthias, 45472 Mülheim an der Ruhr (DE)

(56) Entgegenhaltungen:
- EP-A- 0 631 774
- EP-A- 0 835 897
- EP-A- 0 999 230
- EP-A- 1 544 232
- EP-A- 1 595 909
- WO-A-2005/108449
- DE-A1-102004 034 740
- DE-A1-102005 001 040
- US-A- 4 306 050
- US-A- 5 523 373
- US-A- 5 977 282

## Beschreibung

Gegenstand der Erfindung sind Organopolysiloxan(meth-)acrylat-Partikel, deren Herstellung mittels Suspensionspolymerisation sowie deren Verwendung.

Feine Pulver aus Siliconelastomeren finden bereits Verwendung als Zusatzstoffe in Kosmetika und Toilettenartikeln, z. B. als Mattierungsmittel, als Absorber für Hautfette oder zur Erzeugung eines seidigen Hautgefühls, als Additive zur Verbesserung der mechanischen Eigenschaften von Polymeren und Lacken oder Beschichtungen, z. B. zur Erhöhung der Abrieb- oder Kratzfestigkeit und auch der Schlagzähigkeit, weiterhin als Antiblockmittel zur Verbesserung der Gleiteigenschaften verschiedenster Oberflächen, als Fließ- oder Dispergierhilfsmittel in Pulvern, als Additive in Tonern sowie als mildes Abrasivum in Wasch- und Pflegeformulierungen.

Zur Herstellung solcher Partikel sind mehrere Methoden bekannt. Prinzipiell können irregulär geformte Siliconelastomerpartikel durch Vermahlungsprozesse der jeweiligen Bulk-Elastomere gewonnen werden, jedoch bieten sphäroide oder kugelförmige Partikel in der Regel anwendungstechnische Vorteile, vor allem, wenn eine anprechende Haptik der partikeladditivierten Materialien und Formulierungen gewünscht wird. Üblicherweise werden solche Partikel durch Vernetzungsreaktionen innerhalb von Edukt-Tröpfchen oder Aufwachsen/Aufbringung eines Polymers auf einen Partikelkern hergestellt. Vernetzungsreaktionen können dabei Hydrosilylierungsreaktionen, Kondensationsreaktionen, dehydrogenative Kopplungsreaktionen oder auch radikalische Polymerisationen sein.

Siliconpartikel aus Hydrosilylierungen werden beispielsweise in US 4,761,454, JP 2003301047 und EP 1 074 575 beschrieben, Hydrolyse- und Kondensationsreaktionen zur Herstellung von Siliconpartikeln finden sich in EP 1 130 046, WO 2006/016968, JP 2003002973, US 6,753,399, EP 0 765 896 sowie EP 0 744 432, während US 2004/0156808 eine dehydrogenative Kopplungsreaktion zu diesem Zweck beschreibt. Schließlich werden in DE 10 2004 053 314 über radikalische Polymerisationen erhältliche Copolymere beschrieben.

Die Miniemulsionspolymerisation von Siliconacrylaten zu nanoskaligen Partikeln unter Verwendung eines herkömmlichen Emulgators und eines gängigen molekularen Radikalstarters, wie beispielsweise AIBN, ist literaturbekannt ("Polydimethyl siloxane latexes and copolymers by polymerization and polyaddition in miniemulsion", Katharina Landfester, Ute Pawelzik, Markus Antonietti, Polymer, 46 (2005), 9892-9898). Jedoch liefert der beschriebene Prozess keine mikroskaligen Partikel, welche die gewünschten anwendungstechnischen Eigenschaften besitzen, z. B. ist mit solchen Partikeln nicht das für Personal Care-Anwendungen gewünschte gute Hautgefühl zu erzielen.

Festkörperstabilisierte, wässrige Emulsionen wurden 1907 von S. U. Pickering beschrieben ("Emulsions", Spencer Umfreville Pickering, Journal of the Chemical Society, Transactions (1907), 91, 2001-2021) und gelten als besonders stabil gegenüber Koaleszenz. So beschreibt DE 10 2004 014 704 beispielsweise die Herstellung von Emulsionen, die mit pyrogen erzeugten Partikeln stabilisiert sind. Eine gute Übersicht über die Eigenschaften solcher stabilisierender Feststoffpartikel findet sich in "Particles as surfactants - similarities and differences" von Bernhard P. Binks (Current opinion in colloid & interface science, 7 (2002), 21-41). Zum Stand der Technik gehören auch sogenannte "Janus-Partikel", amphiphile Partikel mit hemisphärisch modifizierter Oberfläche, wie z. B. in FR 2 808 704 beschrieben. Besonders gut geeignet zur Emulsionsstabilisierung sind nanoskalige, vorwiegend anorganische Partikel, z. B. Silica-Partikel, welche als "LUDOX®" in Form wässriger Sole bzw. Dispersionen von der Fa. Grace Davison im Handel erhältlich sind. In US 3,615,972 (1967) wird erstmals die Verwendung von LUDOX®-Partikeln zur Emulsionsstabilisierung von Methylmethacrylat mit nachfolgender Polymerisation beschrieben. Als Mechanismus der stabilisierenden Wirkung wird in der Literatur die Agglomeration der Partikel und die Anreicherung der Agglomerate an der Wasser/Öl-Grenzfläche diskutiert ("The mechanism of emulsion stabilization by small silica (LUDOX®) particles", Helen Hassander, Beatrice Johansson, Bertil Törnell, Colloids and Surfaces, 40, (1989), 93-105).

Die Suspensionspolymerisation von Pickering-Emulsionen schlecht oder nicht wasserlöslicher Edukte muss nach dem bisherigen Stand der Technik mittels eines in der Ölphase gelösten Radikalstarters gestartet werden, der Einsatz wasserlöslicher Radikalstarter führt, beispielsweise mit Styrol als einzigem Monomer, zu unvollständiger Umsetzung und Koagulation ("Pickering stabilized miniemulsion polymerization: Preparation of clay armored latexes", Severine Cauvin, Patrick J. Colver, and Stefan A. F. Bon, Macromolecules 2005, 38, 7887-7889). Nachteilig bei einer solchen Suspensionspolymerisation, welche mit einem molekularen Radikalstarter eingeleitet wird, ist, dass Reaktionsprodukte des Radikalstarters im Polymer verbleiben und sich beispielsweise durch einen störenden Geruch oder auch durch reizende oder toxische Eigenschaften bemerkbar machen können.

Die beschriebenen Verfahren zur Herstellung von Siliconpartikeln umschließen Hydrosilylierung, radikalische Polymerisation, dehydrogenative Kopplung oder Kondensation emulgierter Vorläufer, Sprühprozesse sowie das Einpressen der Vorläufer in geeignete Medien mit anschließender sofortiger Vernetzung.

Die so hergestellten Partikel haben überwiegend den Nachteil, dass sie nicht als rieselfähige Pulver anfallen und somit schwierig handhabbar, d.h. beispielsweise schwer dosierbar und nur aufwändig in den jeweiligen Formulierungen homogenisierbar sind. Weiterhin enthalten sie meist Anteile von Vernetzungskatalysatoren, oft mit Elementen der 8. Nebengruppe des PSE, Emulgatoren und gegebenenfalls weitere Prozesshilfsmittel. In kosmetischen Formulierungen wie auch Reinigungs- und Pflegemitteln ist dies unerwünscht oder zumindest bedenklich.

Ein weiterer Nachteil der gemäß dem Stand der Technik hergestellten Partikel besteht darin, dass polydimethylsiloxan-ähnliche Partikeloberflächen nur schwer modifizierbar sind.

Dies ist aber oftmals gewünscht, um die Partikel an die verschiedenen technischen Erfordernisse anpassen zu können, d.h. beispielsweise ihre Anbindung an diverse Matrices zu ermöglichen oder Einarbeitbarkeit in Formulierungen zu erleichtern bzw. erst möglich zu machen.

Diese Nachteile können teilweise durch Komposit-Partikel überwunden werden. Als Komposit-Partikel werden hier Kern-Hülle-Partikel sowie Partikel, in die zusätzliche Feststoffe eingelagert sind, bezeichnet.

Beispielsweise beschreibt US 4,946,893 (EP 0 319 828) die Herstellung mit anorganischen Partikeln gefüllter Siliconpartikel über eine Hydrosilylierungsreaktion in wässriger Phase und US 5,176,960 beschreibt die Herstellung hochgefüllter mechanisch belastbarer Siliconpartikel über Mischen eines hydrophobierten SiO₂ mit einem Diorganopolysiloxan und anschließender Härtung durch Sprühtrocknung.

Dahingegen erlauben Kern-Hülle-Partikel ggf. gezielte Modifizierungen von Oberflächeneigenschaften, die Einfluss auf die gewünschten anwendungstechnischen Eigenschaften haben.

Je nach Herstellungsverfahren und Verwendung der Kern-Hülle-Partikel kann ihre Korngröße im Nanometer- oder Mikrometer-Bereich liegen. Kern-Hülle-Partikel sind beispielsweise herstellbar nach literaturbekannten Verfahren, so beschreibt die EP 0 661 334 mit einem Organopolysilsesquioxan-Harz oberflächenbeschichtete Siliconpartikel und deren Herstellung, US 2006/0084758 beschreibt die Herstellung von mit kleineren Siliconpartikeln oberflächenmodifizierte Siliconpartikel, und mit SiO₂ aus wässriger Phase nachträglich beschichtete Siliconpartikel finden sich in EP 0 079 322, EP 5 487 624 und EP 0 516 057. Weiterhin beschreibt EP 0 079 322 mit SiO₂ unter Zuhilfenahme einer öligen Phase oberflächenbeschichteter Siliconpartikel. Kern-Schale-Partikel mit Siliconpolymer-Kern und Organopolymer-Hülle werden beschrieben in DE 10 2004 047 708 und DE 10 2004 022 406 (Verwendung in wässrigen Lacken EP 0 882 105, in Pulverlacken EP 0 852 610).

Weiterhin gibt es zahlreiche Schriften, die sich auf Kern-Hülle-Strukturen mit anorganischem Kern und Siliconhülle beziehen, z. B. EP 0 822 232 und EP 0 433 727.

Nachteilig bei diesen Verfahren zur Erzeugung von Kern-Hülle-Partikeln ist, dass es zeit- und ernergieaufwändige, mehrstufige Prozesse sind.

Aufgabe der vorliegenden Erfindung war die Bereitstellung von Silicon(meth-)acrylat-Partikeln, die einen Nachteil oder mehrere Nachteile der Partikel des Standes der Technik nicht aufweisen sowie die Bereitstellung eines alternativen Verfahrens, welches vorzugsweise verfahrensökonomisch vorteilhaft ist. Die hergestellten Partikel sollten vorzugsweise eine kugelige Morphologie aufweisen sowie als Pulver gut rieselfähig sein. Weiterhin sollten die hergestellten Partikel möglichst frei von Emulgatoren, Vernetzungskatalysatoren - vor allem Katalysatoren mit Elementen der 8. Nebengruppe des PSE sowie auch Zinnsalzen - und weiteren Prozesshilfsmitteln sein. Bevorzugte Partikel sollten außerdem eine zusätzliche, nachträgliche und vor allem einfache Modifizierung der Partikeloberflächen erlauben, welche der Kompatibilisierung mit unterschiedlichen Matrices oder Medien bzw. der Anbindung an diese Matrices dienen kann.

Diese Aufgabe wird gelöst durch Suspensionspolymerisation einer wässrigen (ggf. festkörperstabilisierten) Emulsion geeigneter Mono- oder Oligomere bzw. Makromonomere von (meth-)acrylatgruppen-modifizierten Organosiloxanen mittels eines der wässrigen Phase zugegebenen, vorzugsweise wasserlöslichen Radikalstarters.

Überraschenderweise wurde gefunden, dass es gelingt, in einer Suspensionspolymerisation eine wässrige Emulsion von Silicon(meth-)acrylaten und ggf. zugefügten organischen Comonomeren und/oder weiteren Substanzen mit einem in der wässrigen Phase gelösten anorganischen Radikalstarter zu kugeligen Partikeln zu polymerisieren, ohne dass dabei wie im Stand der Technik für konventionelle ungesättigte Monomere (z. B. Styrol, Methylmethacrylat) beschrieben, Koagulation eintritt. Dabei können sowohl Pickering-Emulsionen als auch herkömmliche, durch molekulare Emulgatoren bzw. Tenside stabilisierte Emulsionen zum Einsatz kommen.

Gegenstand der Erfindung ist daher ein Verfahren zur Herstellung von Silicon(meth-)acrylat-Partikeln gemäß Anspruch 1, welches dadurch gekennzeichnet ist, dass eine wässrige Emulsion, enthaltend monomere Silicon(meth-)acrylate und ggf. organische ungesättigte Comonomere sowie die Emulsion stabilisierende Emulgatoren und ein oder mehrere Coemulgatoren, unter Verwendung eines der wässrigen Phase zugegebenen Radikalstarters polymerisiert wird.

Ebenfalls Gegenstand der vorliegenden Erfindung sind so hergestellte Silicon(meth-)acrylat-Partikel sowie deren Verwendung.

Das erfindungsgemäße Verfahren hat den Vorteil, dass beim Polymerisationsstart über einen wasserlöslichen Radikalstarter keine Reaktionsprodukte des Starters im Polymer verbleiben, wie es beispielsweise bei öllöslichen Radikalstartern der Fall ist. Dies ist vorteilhaft für Anwendungen in den Bereichen Kosmetik, Lebensmittelverpackung, Medizinprodukte etc.

Das erfindungsgemäße Verfahren hat außerdem den Vorteil, dass bei der Herstellung der Silicon(meth-)acrylat-Komposit-Partikel über eine Suspensionspolymerisation von Pickering-Emulsionen keine herkömmlichen Emulgatoren nötig sind, welche später in Anwendungen stören könnten und deshalb wieder aufwändig abgetrennt werden müssten.

Außerdem können die erfindungsgemäßen bzw. erfindungsgemäß hergestellten Kern-HülleSilicon(meth-)acrylatpartikel gut rieselfähig sein, so dass für diesen Zweck keine nachträgliche Oberflächenbehandlung mehr nötig ist.

Das erfindungsgemäße Verfahren zur Herstellung von erfindungsgemäßen Silicon(meth-)acrylat-Partikeln sowie deren Verwendung werden nachfolgend beispielhaft beschrieben, ohne dass die Erfindung auf diese beispielhaften Ausführungsformen beschränkt sein soll.

Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer oder Polymer) oder geordnet (Blockoligomer oder Blockpolymer) in diesen Verbindungen vorkommen. Angaben zu Anzahl von Einheiten in solchen Verbindungen sind als Mittelwert, gemittelt über alle entsprechenden Verbindungen zu verstehen.

Das erfindungsgemäße Verfahren zur Herstellung von Silicon(meth-)acrylat-Partikeln umfasst die Schritte
a) Erzeugen einer Emulsion aus Wasser und einer organischen Phase, wobei die organische Phase, end- und/oder seitenständig mit (Meth-)Acrylat-Gruppen modifizierte Organopolysiloxane nach der allgemeinen Formel (I) 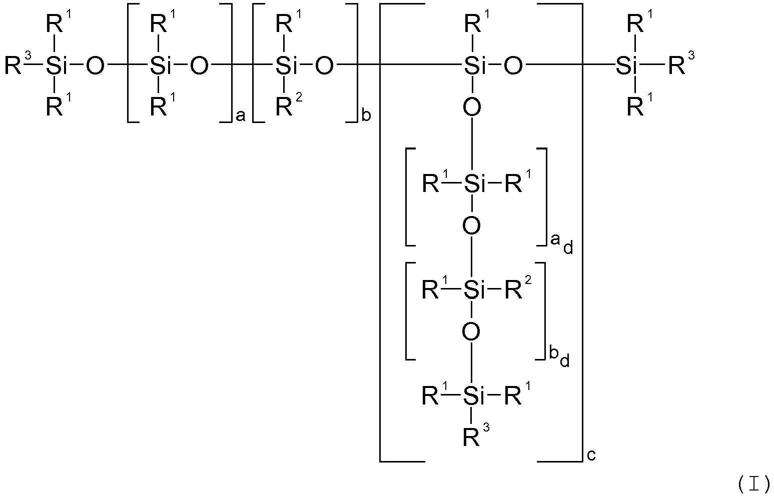 mit
   - R¹: gleiche oder verschiedene Reste, ausgewählt aus linearen oder verzweigten, gesättigten, ein- oder mehrfach ungesättigten, gegebenenfalls eine oder mehrere Ether- oder Esterbrücken enthaltende lineare, cyclische oder verzweigte Alkyl-, Alkoxy-, Polyalkoxy-, Hydroxyalkyl-, Hydroxyalkoxy-, Alkenyl-, insbesondere Vinyl-, Aryl-, Aryloxy-, Hydroxyaryl-, Hydroxyaryloxy-, Alkaryl-, Alkaryloxy-, Hydroxyalkaryl-, Hydroxyalkaryloxy-, Aralkyl-, Aralkoxy-, Hydroxyaralkyl- oder Hydroxyaralkoxy-Resten mit 1 bis 20 C-Atomen, vorzugsweise gleiche oder verschiedene Reste, ausgewählt aus linearen oder verzweigten, gesättigten, ein- oder mehrfach ungesättigten, gegebenenfalls eine oder mehrere Ether- oder Esterbrücken enthaltende lineare, cyclische oder verzweigte Alkyl-, Aryl-, Alkaryl- oder Aralkylresten mit 1 bis 20 C-Atomen, bevorzugt Methyl- oder Phenylreste,
   - R²: gleiche oder verschiedene, an das Si-Atom über eine Si-C-Verknüpfung oder eine Si-O-C-Verknüpfung gebundene, gegebenenfalls eine oder mehrere Ether- oder Esterbrücken enthaltende zweiwertige, ggf. OH-funktionalisierte Kohlenwasserstoffreste, vorzugsweise lineare oder verzweigte, aliphatische, aromatische oder cyclische Kohlenwasserstoffbrücken, die durch ein oder mehrere Ether- und/oder Esterfunktionen unterbrochen sein können und optional ein oder mehrere OH-Funktionen tragen können, mit 1 bis 20 C-Atomen, an welche 1 bis 5 Acrylsäure- und/oder Methacrylsäure-Einheiten und gegebenenfalls Monocarbonsäure-Einheiten mit 2 bis 10 C-Atomen, welche frei von zur Polymerisation befähigten Doppelbindungen sind, über Esterbindungen angebunden sind,
   - R³: gleiche oder verschiedene Reste R¹ oder R²,
   - a: 0 bis 1.000,
   - b: 0 bis 200,
   - c: 0 bis 200, bevorzugt c = 0,
   - a_{d} =: 0 bis 1.000,
   - b_{d}: = 0 bis 200,
   wobei der Index d für c > 0 eine ganze Zahl > 0 ist, mit der Maßgabe, dass wenn b und c = 0 sind, R³ nicht aus der gleichen Gruppe wie R¹ ausgewählt sein darf, oder deren Gemische enthält,
   unter Zugabe zumindest eines Emulgators, vorzugsweise eines Festkörperemulgators (partikulären Emulgators) und optional einen oder mehrerer Coemulgatoren, wobei die organische Phase die innere Phase der Emulsion bildet und
b) Auspolymerisieren der inneren Phase in Gegenwart eines Radikalstarters, welcher der äußeren, wässrigen Phase in einer Konzentration von 0,1 bis 40 Gew.-%, bevorzugt 0,5 bis 25 Gew.-% und besonders bevorzugt 1 bis 10 Gew.-%, bezogen auf die innere Phase zugefügt wird. Das Auspolymerisieren erfolgt also in Form einer Suspensionspolymerisation.

Die Zahlenwerte für a, b und c stellen vorzugsweise statistische Mittelwerte dar. Der Index d ist ein ganzzahliger Indexterm (Laufvariable).

Die in R² genannten Kohlenwasserstoffbrücken können beispielsweise Alkylen-, Alkenylen-, Alkoxylen-, Polyalkoxylen-, Hydroxyalkylen-, Hydroxyalkoxylen-, Arylen-, Aryloxylen-, Hydroxyarylen-, Hydroxyaryloxylen-, Alkylarylen-, Alkaryloxylen-, Hydroxyalkarylen, Hydroxyalkaryloxylen, Aralkylen-, Aralkoxylen-, Hydroxyaralkylen- oder Hydroxyaralkoxylen-Reste sein.

Als Emulgatoren können alle üblichen Emulgatoren eingesetzt werden. Dabei kann es sich um anionische, kationische oder nichtionische oberflächenaktive Substanzen handeln.

Typische Emulgatoren sind z. B. Alkylsulfate, vorzugsweise mit einer Kettenlänge von 10 bis 18 C-Atomen, Alkyl- und Arylethersulfate, vorzugsweise mit 10 bis 24 C-Atomen im hydrophoben Rest und mit bevorzugt bis zu 40 Ethylenoxid- oder Propylenoxid-Einheiten, Alkyl- und alkylarylsulfonate mit vorzugsweise 10 bis 24 C-Atomen, Alkyldiphenyloxiddisulfonate, Ölsäuresulfonate, Ester und Halbester der Sulfobernsteinsäure mit einwertigen Alkoholen oder Alkylphenolen, Alkyl- und Alkenylcarboxylate mit vorzugsweise einer Kettenlänge von 10 bis 18 C-Atomen, Alkylpolyglykolether und Alkylarylpolyglykolether mit vorzugsweise jeweils 4 bis 40 Ethylenoxideinheiten, Alkyl- und Alkenylalkohole mit vorzugsweise 12 bis 20 C-Atomen, ethoxylierte Alkyl- und Alkenylalkohole mit vorzugsweise 12 bis 20 C-Atomen und ethoxylierte Alkylphenole. Für kosmetische Anwendungen eignen sich insbesondere Emulgatorsysteme, die üblicherweise zur Emulgierung von Siliconölen dienen, wie sie beispielsweise von der Evonik Goldschmidt GmbH unter den Namen TEGO® Care PL 4 oder ABIL® Care 85 angeboten werden. Insbesondere können aus kosmetischen Anwendungen bekannte Emulgatoren und Tenside eingesetzt werden, wie sie etwa in DE 10 2005 011785 A1 aufgeführt sind.

Es kann vorteilhaft sein, wenn in Schritt a) eine festkörperstabilisierte Emulsion erzeugt wird. Dazu können als Emulgatoren partikuläre, vorzugsweise in mindestens einer Dimension nanoskalige Partikel oder nanostrukturierte Partikel oder Nanoobjekte, die besonders bevorzugt ausgewählt sind aus der Gruppe der Metalloxide, Mischoxide, Nitride, Hydroxide, Carbonate, Silikate, Siliconharze, Silicone und/oder organischen Polymere, die vorzugsweise zumindest (teil-)hydrophobiert sind, z. B. mit mindestens einer Verbindung aus der Gruppe der Silane, Siloxane, quaternären Ammoniumverbindungen, kationischen Polymere und Fettsäuren oder deren Anionen, eingesetzt werden. Durch den Einsatz von partikulären Emulgatoren können mit dem erfindungsgemäßen Verfahren Siliconacrylat-Komposit-Partikel, insbesondere solche des Kern-Hülle-Typs, hergestellt werden. Diese weisen im Kern das polymerisierte Siliconacrylat und als Hülle die partikulären Emulgatoren auf. Unter Nanoobjekten werden im Rahmen der vorliegenden Erfindung Materialien verstanden, die entweder in ein, zwei oder drei äußeren Dimensionen nanoskalig sind, vorzugsweise zumindest eine Dimension eine Größe von 1 bis 100 nm aufweist, wie z. B. Nanoplättchen, Nanostäbchen und Nanopartikel. Unter nanostrukturierten Partikeln werden in der vorliegenden Erfindung Materialien bzw. Partikel verstanden, die eine innere nanoskalige Struktur aufweisen. Typische Vertreter sind z. B. Aggregate und Agglomerate von Nanoobjekten.

Besonders bevorzugte partikuläre Emulgatoren weisen eine mittlere Primärpartikelgröße in mindestens einer Dimension von < 1.000 nm, bevorzugt < 500 nm und besonders bevorzugt von 1 bis 100 nm auf. Die Primärpartikelgröße kann auf bekannte Weise bestimmt werden. Vorzugsweise wird die Primärpartikelgröße durch die optische Auswertung einer durch Transmissionselektronenmikroskopie erstellten Aufnahme bestimmt.

Die partikulären Emulgatoren können in dem erfindungsgemäßen Verfahren als solche oder in Form von Dispersionen oder Solen, insbesondere wässrigen Dispersionen oder Solen eingesetzt werden.

Insbesondere bei der Verwendung von partikulären Emulgatoren kann es vorteilhaft sein, wenn in Schritt a) des erfindungsgemäßen Verfahrens die Herstellung der Emulsion unter Zugabe eines oder mehrerer Coemulgatoren durchgeführt wird. Als Coemulgatoren können in dem erfindungsgemäßen Verfahren insbesondere solche Verbindungen eingesetzt werden, die mit den Festkörperemulgator-Partikeln in Wechselwirkung treten, vorzugsweise solche, die zu hydrophobierenden Festkörperemulgator-Partikel aufziehen. In dem erfindungsgemäßen Verfahren können als Coemulgatoren insbesondere Verbindungen ausgewählt aus der Gruppe der kationischen Tenside eingesetzt werden. Als kationische Coemulgatoren können insbesondere kationische Ammoniumverbindungen eingesetzt werden. Solche Verbindungen sind z. B. unter den Handelsnamen VARISOFT® 470 P, VARISOFT® TC-90, VARISOFT® 110, AROSURF® TA-100, ADOGEN® 442-100 P, ADOGEN® 432, ADOGEN® 470, ADOGEN® 471, ADOGEN® 464, VARIQUAT® K 300, VARIQUAT® B 343, VARIQUAT® 80 ME, REWOQUAT® 3690, REWOQUAT® WE 15, REWOQUAT® WE18, REWOQUAT® WE 28 oder REWOQUAT® CR 3099 bei der Evonik Goldschmidt GmbH erhältlich. Bevorzugt wird in dem erfindungsgemäßen Verfahren Cetyltrimethylammoniumbromid oder -chlorid als kationischer Coemulgator eingesetzt.
In dem erfindungsgemäßen Verfahren werden als Silicon(meth-)acrylate der Formel (I) vorzugsweise solche eingesetzt, bei denen mehr als 70 %, besonders bevorzugt mehr als 90 %, ganz besonders bevorzugt alle der Reste R¹ in Formel (I) Methylgruppen sind.

Die Reste R² in der allgemeinen Formel (I) sind vorzugsweise ausgewählt aus der Gruppe der Reste mit den Formeln (IIa) bis (IIj) 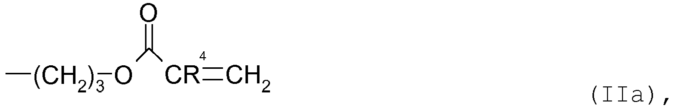 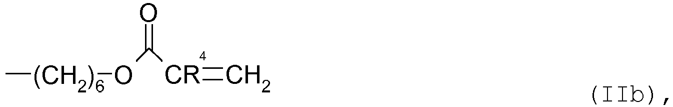 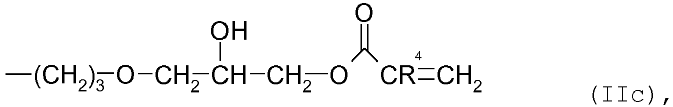 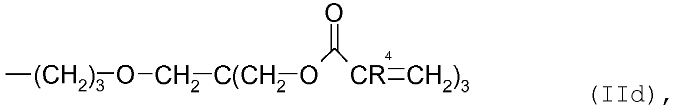 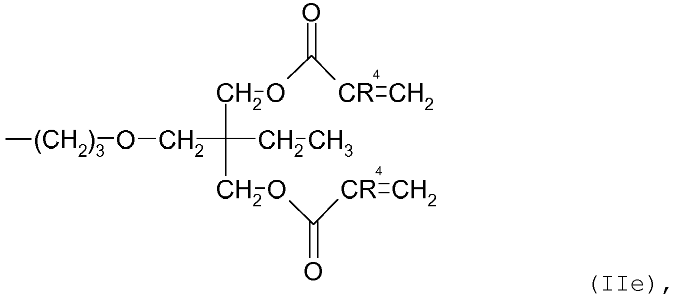 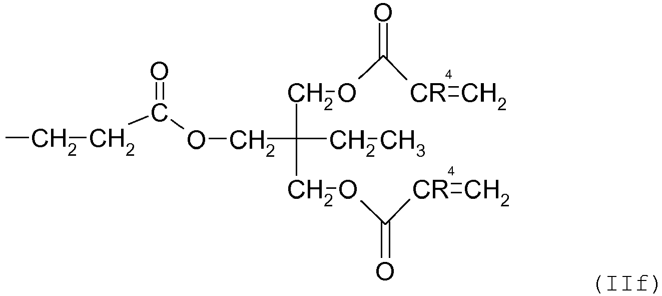 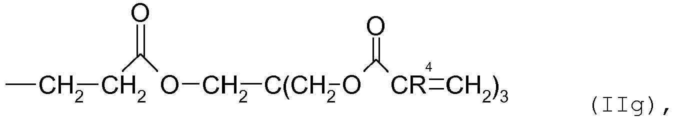 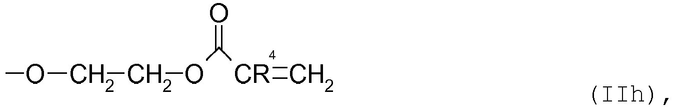 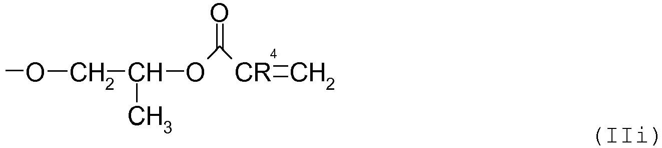 und/oder

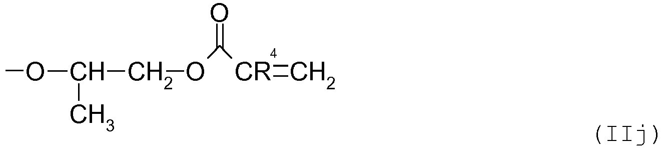

Besonders bevorzugt ist, wenn R² einer der genannten Reste und der Rest R⁴ Wasserstoff oder eine Methylgruppe ist. Ganz besonders bevorzugt werden in dem erfindungsgemäßen Verfahren solche Silicon(meth-)acrylate der Formel (I) eingesetzt, bei denen die Reste R¹ zu den oben genannten Anteilen Methylgruppen sind, die Reste R² ausgewählt sind aus den Resten der Formeln IIa bis IIj und der Rest R⁴ Wasserstoff oder eine Methylgruppe ist.

Es kann vorteilhaft sein, wenn Silicon(meth-)acrylate der Formel (I) eingesetzt werden, bei denen a einen Wert von 0 bis 500, vorzugsweise von 2 bis 250, einnimmt.

Ebenso kann es vorteilhaft sein, wenn Silicon(meth-)acrylate der Formel (I) eingesetzt werden, bei denen b einen Wert von 0 bis 100, vorzugsweise 1 bis 50 und bevorzugt 3 bis 25 einnimmt.

Außerdem kann es vorteilhaft sein, wenn Silicon(meth-)acrylate der Formel (I) eingesetzt werden, bei denen c einen Wert von 0 bis 100, vorzugsweise von 0 bis 50, bevorzugt 0 einnimmt.

Besonders vorteilhaft kann es sein, wenn Silicon(meth-)acrylate der Formel (I) eingesetzt werden, bei denen a einen Wert von 0 bis 500, vorzugsweise von 2 bis 250, einnimmt, b einen Wert von 0 bis 100, vorzugsweise 1 bis 50 und besonders bevorzugt 3 bis 25, einnimmt und c einen Wert von 0 bis 100, vorzugsweise von 0 bis 50, bevorzugt 0 einnimmt und a_{d} und b_{d} entsprechend a und b definiert sind.

Als Silicon(meth-)acrylate können insbesondere die bei der Evonik Goldschmidt GmbH erhältlichen Produkte TEGO® RC 705, 706, 708, 709, 710, 711, 712, 715, 716, 719, 726, 902, 2015 oder TEGO® Rad 2100, 2200 N (Siliconpolyetheracrylat), 2250 (Siliconpolyetheracrylat), 2300 (Siliconpolyetheracrylat), 2350 (Siliconpolyetheracrylat), 2500 (acrylmodifiziertes Polydimethylsiloxan), 2600 (acrylmodifiziertes Polysiloxan), 2650 (acrylmodifiziertes Polysiloxan) oder 2700 (acrylmodifiziertes Polysiloxan) eingesetzt werden.

Im erfindungsgemäßen Verfahren können die Silicon(meth-)acrylate der Formel (I) einzeln oder als Mischungen, insbesondere als statistische Mischungen eingesetzt werden. Vorzugsweise werden in dem erfindungsgemäßen Verfahren Mischungen von Silicon(meth-)acrylaten der Formel (I) eingesetzt, in denen die Silicon(meth-)acrylate sich bezüglich ihrer Struktur und/oder ihrem Molekulargewicht unterscheiden.

Es kann vorteilhaft sein, wenn der organischen Phase vor Schritt b) Comonomere, insbesondere ethylenisch oder vinylisch ungesättigte Gruppen aufweisende Comonomere zugefügt werden. Solche Comonomere können z. B. ein- oder mehrfach (meth-)acrylierte, organische Mono- oder Oligomere, wie sie beispielsweise auch unter den Gruppennamen LAROMER® (BASF AG), EBECRYL® (Cytec Surface Specialties)oder DESMOLUX® (Bayer Material Science) im Handel sind, sein. Unter ethylenisch ein- oder mehrfach ungesättigten organischen Mono- oder Oligomeren sollen auch ethylenisch ungesättigte, bevorzugt vinylische Gruppen tragende Organopolysiloxane verstanden werden. Vorzugsweise erfolgt die Zugabe der weiteren Comonomere vor Schritt a).

Die organischen Comonomere können nach abgeschlossener Polymerisation in Schritt b) sowohl in das Polysiloxan-(meth)acrylat-Netzwerk kovalent einreagiert sein als auch als eigenständiges Netzwerk vorliegen. Ebenso sind Mischformen dieser beschriebenen Grenzfälle möglich und Teil dieser Erfindung.

In dem erfindungsgemäßen Verfahren können der organischen, inneren Phase vor Schritt b), vorzugsweise vor Schritt a) kationische Gruppen enthaltende Organopolysiloxane, die auch reaktive Gruppen, wie z. B. vinylische Gruppen, ethylenisch ungesättigte Gruppen oder Expoxy-Gruppen, tragen können, zugefügt werden. Vorzugsweise werden die kationische Gruppen enthaltenden Organopolysiloxane, die auch reaktive Gruppen, wie z. B. vinylische Gruppen, ethylenisch ungesättigte Gruppen oder Expoxy-Gruppen, tragen können, in einer Konzentration von bis zu 25 Gew.-%, bevorzugt bis zu 10 Gew.-%, besonders bevorzugt bis zu 5 Gew.-%, bezogen auf die Gesamtreaktionsmasse zugefügt.

Auf diese Weise sind auch kationische Partikel erhältlich, durch die sowohl eine gute Haftung auf speziellen Substraten, als auch eine elektrostatische oder elektrosterische Stabilisierung von Dispersionen erreicht werden kann. Beispiele für solche kationischen Gruppen tragenden Organopolysiloxane sind z. B. ABIL® Quat 3272 und ABIL® 3474 (Evonik Goldschmidt GmbH).

Optional können der organischen Phase in Schritt a) weitere Komponenten zugefügt werden. Die weiteren Komponenten können in der organischen Phase oder der Mischung in Schritt a) gelöst oder dispergiert sein. Solche weiteren Komponenten können funktionelle Komponenten oder nichtfunktionelle Komponenten sein. Als weitere Komponenten können insbesondere eindispregierbare Feststoffe, wie z. B. anorganische Partikel und/oder Fasern, wie z. B. solche der Metalloxide, Mischoxide, Nitride, Hydroxide, Carbonate, Silikate, Pigmente, Ruße, Elemente oder Legierungen, und/oder organische Partikel und/oder Fasern, wie z. B. solche aus Siliconharzen, Siliconen oder organische Polymere oder Biopolymeren, eingesetzt werden, vorzugsweise mit der Maßgabe, dass die Füllstoffe von den eingesetzten Emulgatoren verschieden sind. Eindispergierbare Feststoffe können beispielsweise Fällungskieselsäure, Diatomeenerde (Kieselgur), pyrogene Kieselsäure, Quarzmehl, Titandioxid, Zinkoxid, Ceroxid, Eisenoxid, Ruß, Graphit, Kohlenstoff-Nanoröhren oder -fasern, Alumosilikate, Erdalkalicarbonate, Aluminiumtrihydroxid, Magnesiumdihydroxid bzw. andere aus dem Stand der Technik bekannte und übliche Feststoffe sowie jede der genannten Substanzen nach Oberflächenmodifizierung mit Organosiliziumverbindungen wie Trimethylchlorsilan, Hexamethyldisilazan, (Meth-)Acryloxypropyltrialkoxysilanen, Aminopropyltrialkoxysilanen, Polydimethylsiloxanen, Polysiloxanen, die Si-H-Gruppen tragen, oder reinen Carbonsäuren, Chelatbildnern oder Fluoropolymeren. Diese Feststoffe können beispielsweise als Füllstoffe zur Erzielung bestimmter mechanischer Eigenschaften, als UV-Schutzmittel, als Pigmente, als Antistatik-Zusätze oder zur Erzielung ferromagnetischer Eigenschaften dienen.

In dem erfindungsgemäßen Verfahren kann die organische Phase auch Substanzen beinhalten, welche ggf., vorzugsweise über einen längeren Zeitraum hinweg, aus den Partikeln freigesetzt werden können. Solche Substanzen können z. B. kosmetische Öle und Wirkstoffe, Duftstoffe, pharmazeutische Wirkstoffe, antimikrobielle Wirkstoffe, auch zum Beispiel Silber und silberhaltige Verbindungen, sowie Farb- und Konservierungsstoffe sein.
Es kann vorteilhaft sein, wenn in Schritt a) des erfindungsgemäßen Verfahrens eine Emulsion erzeugt wird, deren mittlere Tröpfchengröße von 0,01 bis 1.000 µm, bevorzugt 0,1 bis 500 µm und besonders bevorzugt von 1 bis 100 µm eingestellt wird.

Die Tröpfchengröße kann unter Zuhilfenahme der Lichtmikroskopie (bis ca. 1 µm als untere Grenze) durch Ausmessen des jeweils kleinsten und größten Tröpfchendurchmessers im Sichtfeld abgeschätzt werden, es sollten sich dabei mindestens 10 x 10 Tropfen im Sichtfeld befinden. Weiterhin ist es möglich, die Tröpfchengrößenverteilungen durch die dem Fachmann geläufigen Methoden der statischen und der dynamischen Lichtstreuung zu bestimmen. Dies gilt auch für Dispersionen auspolymerisierter Partikel, darüber hinaus ist die Partikelgrößenbestimmung durch elektronenmikroskopische Aufnahmen über REM oder TEM bestimmbar und dem Fachmann geläufig.

Vorzugsweise wird die Emulsion in Schritt a) durch Durchleiten des Gemisches enthaltend organische und wässrige Phase durch und Dispergieren des Gemisches in wenigstens einer Interaktionskammer, vorzugsweise mit einer Kapillardicke (Innendurchmesser) von 50 bis 500 µm, und vorzugsweise bei einem Druck von 50 bis 1.000 bar, bevorzugt 100 bis 800 bar, besonders bevorzugt 200 bis 600 bar und anschließende Entspannung des Gemisches auf Umgebungsdruck, z. B. in ein Auslass-Reservoir, hergestellt. Dabei wird vorzugsweise eine der oben genannten bevorzugten Tröpfchengrößen eingestellt. Es kann vorteilhaft sein, wenn zwei oder mehr in Reihe geschaltete Interaktionskammern eingesetzt werden. Auf diese Weise kann die gewünschte Tröpfchengröße leichter eingestellt werden. Die Herstellung von Emulsionen in Interaktionskammern wird ausführlich in US 2004-0063818 bzw. DE 100 11 564 beschrieben, auf die ausdrücklich verwiesen wird. Ein geeignetes Gerät zur Herstellung der Emulsionen wird beispielsweise unter dem Namen Mikrofluidizer von der Firma Microfluidics angeboten.

Um eine Emulsion mit Tröpfchengrößen im bevorzugten Bereich zu erhalten, deren Tröpfchen vorzugsweise eine kugelige Morphologie aufweisen, kann es bei der Zugabe von Coemulgatoren vorteilhaft sein, den Coemulgator bzw. die Coemulgatoren erst zuzugeben, nachdem in einem Teilschritt a1) eine Voremulsion V1 hergestellt wurde. Diese Voremulsion V1 z. B. kann dadurch erhalten werden, dass eine Mischung aus Silicon(meth-)acrylat der Formel (I), Wasser und Emulgator, vorzugsweise partikulärem Emulgator und besonders bevorzugt nanopartikulärem SiO₂ und ganz besonders bevorzugt LUDOX® SM-AS der Fa. Grace Davison unter Aufbringung starker Scherkräfte, wie dies z. B. mit einem Rotor-Rotor-System möglich ist, emulgiert wird. Ein geeignetes Rotor-Rotor-System wird z. B. als Co-Twister Homogenizer von der Firma Symex angeboten.

Bei der Herstellung der Voremulsion V2 wird der Voremulsion V1 in Schritt a2) der Coemulgator zugegeben. Die Coemulgatoren können als Reinstoff oder in Form einer Lösung, z. B. einer wässrigen Lösung zugegeben werden. Durch die Zugabe des Coemulgators zur Voremulsion V1 kann die Tröpfchengröße der in der in der Voremulsion V1 enthaltenen Tropfen quasi eingefroren werden. Durch den Zeitpunkt der Zugabe des Cotensids kann somit Tröpfchengrößenverteilung eingestellt werden. Unter anderem durch die Zugabe-Menge von Emulgator und Coemulgator kann die Tröpfchengrößenverteilung der Emulsion voreingestellt werden. Vorzugsweise beträgt das Gewichts-Verhältnis von partikulärem Emulgator zu Coemulgatoren von 100 zu 1 bis 1 zu 1, bevorzugt von 50 zu 1 bis 3 zu 1.

Die in Schritt a2) erhaltene Voremulsion V2 wird anschließend in Schritt a3) in einem Homogenisator mit Interaktionskammer dispergiert. Vorzugsweise wird die Emulsion in Schritt a) durch Durchleiten des Gemisches enthaltend organische und wässrige Phase durch und Dispergieren des Gemisches in wenigstens einer Interaktionskammer, vorzugsweise mit einer Kapillardicke (Innendurchmesser) von 50 bis 500 µm, und vorzugsweise bei einem Druck von 50 bis 1.000 bar, bevorzugt 100 bis 800 bar, besonders bevorzugt 200 bis 600 bar und anschließende Entspannung des Gemisches auf Umgebungsdruck, z. B. in ein Auslass-Reservoir. Dabei wird vorzugsweise eine der oben genannten bevorzugten Tröpfchengrößen eingestellt. Es kann vorteilhaft sein, wenn zwei oder mehr in Reihe geschaltete Interaktionskammern eingesetzt werden. Auf diese Weise kann die gewünschte Tröpfchengröße besonders einfach eingestellt werden. Ein geeigneter Homogenisator wird beispielsweise unter dem Namen Mikrofluidizer von der Firma Microfluidics angeboten.

Bevorzugt werden in Schritt a3) des erfindungsgemäßen Verfahrens Interaktionskammern eingesetzt, von denen zumindest eine eine Kapillardicke von 100 bis 300 µm aufweist. Besonders bevorzugt werden in Schritt a) des erfindungsgemäßen Verfahrens Interaktionskammern eingesetzt, von denen mindestens eine, vorzugsweise alle, mindestens einen Umlenkknick aufweisen.

Durch die Durchführung der Teilschritte a1) bis a3) und die Verwendung eines Homogenisators mit Interaktionskammer in Teilschritt a3) lassen sich besonders einfach kugelige Tröpfchen mit einer gewünschten Tröpfchengrößenverteilung herstellen.

Die Polymerisation in Schritt b) wird durch den/das der Wasserphase zugefügte/n Radikalstarter bzw. -startersystem initiiert. Der Verfahrensschritt b) kann bei erhöhter Temperatur stattfinden, wird jedoch vorzugsweise bei Raumtemperatur durchgeführt. Vorzugsweise wird der Verfahrensschritt b) unter Rühren durchgeführt. Ansonsten kann die Polymerisation in Schritt b) wie im Stand der Technik beschrieben auf herkömmliche Weise durchgeführt werden.

Als Radikalstarter können übliche als Radikalstarter geeignete Verbindungen eingesetzt werden. Mögliche Radikalstarter können beispielsweise Peroxodisulfate, wie z. B. Ammonium- oder Kaliumperoxodisulfat, Wasserstoffperoxid, Alkylhydroperoxide wie t-Butylhydroperoxid oder Ammonium- oder Kaliumperoxodiphosphat sein. Die Initiierung kann in diesen Fällen beispielsweise durch Temperaturerhöhung erfolgen. Als Radikalstarter können vorzugsweise auch Redox-Systeme eingesetzt werden, welche auch bei niedrigen Temperaturen, bevorzugt bei Temperaturen von < 70 °C, bevorzugt < 45 °C und besonders bevorzugt < 30 °C, gegebenenfalls in Kombination mit katalytisch zersetzend wirkenden Schwermetallsalzen, wie beispielsweise Kupfer- oder Eisensalzen, arbeiten. Bevorzugte als Radikalstarter eingesetzte Redox-Systeme können beispielsweise Peroxodisulfate, wie Ammonium- oder Kaliumperoxodisulfat, Peroxodiphosphate, wie Ammonium- oder Kaliumperoxodiphosphat, Wasserstoffperoxid oder Alkylhydroperoxide, wie t-Butylhydroperoxid, in Kombination mit zumindest einem Reduktionsmittel wie beispielsweise Alkalihydrogensulfite, wie Natriumhydrogensulfit, Alkalidithionite, wie Natriumdithionit, Natriumformaldehydsulfoxylat oder auch Ascorbinsäure sein. Gegebenenfalls kann es vorteilhaft sein, wenn die Radikalstarter in Kombination mit Radikalüberträgern eingesetzt werden. Bevorzugte Radikalüberträger können z. B. Acetylaceton, Aceton oder ähnliches sein. Solche Systeme sind als Radikalstarter gut bekannt und im Bereich der Emulsionspolymerisation Stand der Technik. Zusätzlich können solchen Radikalstartern, insbesondere solchen auf Basis eines Redox-Systems, Puffersysteme hinzugefügt werden, um pH-Wert-Änderungen durch beispielsweise Bildung saurer Gruppen, beispielsweise Hydrogensulfat-Gruppen, abzufangen. Solche Puffersysteme, beispielsweise Carbonat- oder Phosphat-Puffer, sind ebenfalls lange bekannt und Stand der Technik. Bevorzugt werden Phosphat-Puffer eingesetzt, besonders bevorzugt sind Puffer, die den pH-Wert im Bereich um pH 7 stabilisieren, beispielsweise Dialkalihydrogenphosphat, insbesondere Dikaliumhydrogenphosphat bei der Entstehung saurer Gruppen, beispielsweise von Hydrogensulfat, oder die Kombination aus Dialkalihydrogenphosphat, wie Dikaliumhydrogenphosphat, und Alkalidihydrogenphosphat, wie Kaliumdihydrogenphosphat.

Nach der Durchführung des Polymerisationschrittes b) kann es vorteilhaft sein, die erhaltenen Partikel aus der Suspension abzutrennen. Dazu kann z. B. das Wasser nach üblichen Methoden, beispielsweise durch Filtrieren oder Zentrifugieren, entfernt werden. Um den Trocknungsvorgang zu beschleunigen kann es vorteilhaft sein, die Partikel z. B. mit Ethanol zu waschen.

Es kann vorteilhaft sein, wenn die Partikel nach der Synthese oberflächenmodifiziert werden. Die Oberflächenmodifizierung kann nach den üblichen Methoden erfolgen. Handelt es sich bei den Partikeln um Kern-Hülle-Partikel und weist die Hülle Halmetall-/Metalloxide, insbesondere SiO₂ auf, so kann die Oberfläche mit dem Fachmann bekannten Verfahren z. B. mit Trimethylchlorsilan, Dimethyldichlorsilan oder Hexamethydisilazan oder weiteren funktionellen Silanen, auch α-funktionellen Silanen, Carbonsäuren etc. modifiziert werden, um funktionelle Partikel zu erzeugen. Ebenso ist die Belegung der Oberfläche mit anorganischen Verbindungen und Elementen, wie beispielsweise mit Silber, möglich. Auf diese Weise werden mikrobizide Partikel erhalten.

Besonders vorteilhaft kann es sein, wenn das Modifizierungsmittel mindestens eine funktionelle Gruppe aufweist, die mit der zu modifizierenden Oberfläche eine kovalente, ionische oder koordinative Bindung oder Wasserstoffbrückenbindungen eingehen kann. Es kann sich bei diesen funktionellen Gruppen beispielsweise um Carbonsäuregruppen, Säurechloridgruppen, Estergruppen, Nitril- und Isonitrilgruppen, OH-Gruppen, SH-Gruppen, Epoxidgruppen, Anhydridgruppen, Säureamidgruppen, primäre, sekundäre und tertiäre Aminogruppen, Si-OH-Gruppen, hydrolysierbare Reste von Silanen (Si-OR) oder CH-acide Gruppierungen wie z. B. in β-Dicarbonylverbindungen, beispielsweise Acetylaceton, 2,4-Hexandion, 3,5-Heptandion, Diacetyl oder Acetessigsäure, handeln. Ebenso können auch mehr als eine derartige Gruppe in dem Modifizierungsmittel vorhanden sein, wie z. B. in Betainen, Aminosäuren, beispielsweise Glycin, Alanin, β-Alanin, Valin, Leucin, Isoleucin, Arginin und Aminocapronsäure, sowie in EDTA. Carbonsäuren zur Oberflächenmodifizierung sind beispielsweise Fettsäuren, Ameisensäure, Essigsäure, Propionsäure, Buttersäure, Pentansäuren, Hexansäure, Acrylsäure, Adipinsäure, Bernsteinsäure, Fumarsäure, Itaconsäure, Stearinsäure, Hydroxystearinsäure, Ricinolsäure und Polyethercarbonsäuren und deren entsprechende Anhydride, Chloride, Ester und Amide, beispielsweise Methoxyessigsäure, 3,6-Dioxaheptansäure und 3,6,9-Trioxadecansäure sowie die entsprechenden Säurechloride, -ester und -amide.

Neben der mindestens eine funktionelle Gruppe, die mit der Oberfläche des Kern-Hülle-Siliconpartikels eine Bindung eingehen kann, kann das Modifizierungsmittel zusätzlich weitere Reste aufweisen, welche die Eigenschaften des Partikels modifizieren. Solche Reste, oder auch Teile davon, können beispielsweise hydrophob oder hydrophil sein oder eine oder mehrere funktionelle Gruppen tragen, um auf diese Weise die Siliconpartikel kompatibel zum umgebenden Medium zu machen, sie zu inertisieren oder reaktionsfähig zu machen, was auch eine Anbindung an die umgebende Matrix mit einschließt. Diese funktionellen Gruppen können beispielsweise ausgewählt sein aus dem Bereich der Alkyl-, Aryl-, Alkaryl-, Aralkyl-, Fluoroalkyl-, Hydroxy-, Alkoxy-, Polyalkoxy-, Epoxy-, Acryloxy-, Methacryloxy-, Acrylat-, Methacrylat-, Carboxy- Amino-, Sulfonyl-, Sulfat-, Phosphat-, Polyphosphat-, Phosphonat-, Amid-, Sulfid-, Hydrogensulfid-, Halogenalkyl-, Halogenaryl- und Acylgruppen.

Wird die Oberflächenmodifizierung mit Silanen durchgeführt, so können bevorzugt hydrolysierbare Organosilane eingesetzt werden, die zusätzlich mindestens einen nicht hydrolysierbaren Rest aufweisen. Solche Silane werden durch die allgemeine Formel (III)

RₙSiX₍₄₋ₙ₎ (III)

dargestellt, mit
R = gleiche oder verschiedene nicht hydrolysierbare Gruppen,
X = gleiche oder verschiedene hydrolysierbare Gruppen oder Hydroxygruppen und
n = 1, 2, 3 oder 4.

In der allgemeinen Formel (III) können die hydrolysierbaren Gruppen X beispielsweise H, Halogen- (F, Cl, Br, I), Alkoxy- (bevorzugt Methoxy-, Ethoxy-, i-Propoxy-, n-Propoxy- oder Butoxy-), Aryloxy- (bevorzugt Phenoxy-), Acyloxy- (bevorzugt Acetoxy- oder Propionyloxy-), Acyl- (bevorzugt Acetyl-), Amino-, Monoalkylamino- oder Dialkylamino-Gruppen sein. Weiterhin kann es sich in der allgemeinen Formel (III) bei den nicht hydrolysierbaren Resten R sowohl um Reste mit oder ohne funktionelle Gruppen handeln. So kann R in der allgemeinen Formel (III) ohne funktionelle Gruppen beispielsweise ein Alkyl-, Alkenyl-, Alkinyl-, Aryl-, Alkylaryl- oder Aralkyl-Rest sein. Die Reste R und X können ggf. einen oder mehrere übliche Substituenten, wie beispielsweise Halogen oder Alkoxy aufweisen. Bei Resten nach der allgemeinen Formel (III) mit einer funktionellen Gruppe kann die funktionelle Gruppe beispielsweise ausgewählt werden aus dem Bereich der Epoxid- (z.B. Glycidyl- oder Glycidyloxy-), Hydroxy-, Ether-, Amino-, Monoalkylamino-, Diaklyamino-, ggf. substituierten Anilino-, Amid-, Carboxy-, Acryl-, Methacryl-, Acryloxy-, Methacryloxy-, Mercapto-, Cyano-, Alkoxy-, Isocyanato-, Aldehyd-, Alkylcarbonyl-, Säureanhydrid-, Phosphat- und Polyphosphat-Gruppen. Diese funktionellen Gruppen können über Alkylen-, Alkenylen- oder Arylen-Brückengruppen, die durch Sauerstoff oder NH-Gruppen unterbrochen sein können, an das Siliciumatom gebunden sein. Diese zweiwertigen Brückengruppen und ggf. vorliegende Substituenten, wie bei Alkylaminogruppen, können aus den entsprechenden einwertigen Alkyl-, Alkenyl-, Aryl-, Aralkyl- und Alkaryl-Resten abgeleitet werden. Natürlich kann der Rest R auch mehr als eine funktionelle Gruppe aufweisen. Nicht hydrolysierbare Reste R nach der allgemeinen Formel (III) mit funktionellen Gruppen können ausgewählt werden aus dem Bereich der Glycidyl- oder Glycidyloxyalkylen-Reste, wie beispielsweise β-Glycidyloxyethyl, γ-Glycidyloxypropyl, δ-Glycidyloxypropyl, ε-Glycidyloxypentyl, ω-Glycidyloxyhexyl oder 2-(3,4-Epoxycyclohexyl)ethyl, der Methacryloxyalkylen- und Acryloxyalkylen-Reste, wie beispielsweise Methacryloxymethyl, Acryloxymethyl, Methacryloxyethyl, Acryloxyethyl, Methacryloxypropyl, Acryloxypropyl, Methacryloxybutyl oder Acryloxybutyl, und dem 3-Isocyanatopropyl-Rest.

Weiterhin ist auch der Einsatz von Silanen mit zumindest teilweise fluorierten Alkylresten möglich, beispielsweise 3,3,4,4,5,5,6,6,7,7,8,8,8-Tridecafluorooctyl- oder 3,3,3-Trifluoropropylgruppen.

Wird die Hülle der Silicon(meth-)acrylat-Partikel aus SiO₂ gebildet, trägt sie im alkalischen, neutralen und auch noch schwach sauren pH-Bereich eine negative Ladung, da die Festkörperstabilisatorpartikel dort ein negatives ζ-Potential aufweisen. Dies erlaubt eine Modifizierung mit kationischen Substanzen bzw. kationischen Polymeren, welche bis zu einer Umladung der Partikel geführt werden kann. Als Beispiele für solche kationischen Substanzen können Polydimethyldiallylammoniumchlorid (PDADMAC), Chitosan, quaternierte Zellulosederivate (beispielsweise Polyquaternium-10), quaternäre (Organo-)Polysiloxane (beispielsweise Quaternium-80, wie z. B. ABIL® Quat 3272 oder ABIL® Quat 3474 der Evonik Goldschmidt GmbH) oder auch modifizierte Polyharnstoffstrukturen wie in Ralumer 11 der Fa. Raschig dienen. Eine solche Modifizierung erlaubt beispielsweise ein Anhaften der so modifizierten Partikel an negativ geladenen Oberflächen, wie sie bei vielen textilen Fasern, Haut oder Haaren zu finden ist.

Es ist ebenso möglich, insbesondere bei oxidischen Partikeln, wie z. B., colloidalem Silica, wie es z. B. von Grace Davison als LUDOX® erhältlich ist, eine Oberflächenmodifizierung mit Siloxanen und Organopolysiloxanen durchzuführen. Dies kann geschehen durch den Einsatz von mit Trimethylsiloxy-Gruppen endverkappten Dimethylpolysiloxanen, cyclischen Dimethylpolysiloxanen, α,ω-Dihydroxypolydimethylsiloxanen, cyclischen Methylphenylsiloxanen, mit Trimethylsiloxy-Gruppen endverkappten Methylphenylpolysiloxanen oder von mit Trimethylsiloxy-Gruppen endverkappten Dimethylsiloxan-Methylphenylsiloxan-Copolymeren, ggf. in der Gegenwart eines geeigneten Katalysators (beispielsweise Ammoniumcarbamat oder Alkylhydroxiden) und ggf. auch erhöhten Temperaturen.

Die Oberflächenmodifizierung mit Polysiloxanen oder Organopolysiloxanen kann kovalent aber auch adsorptiv erfolgen, Beispiele solcher Substanzklassen sind end- und/oder kammständig mit Polyether- oder Polyesterketten modifizierte Organopolysiloxane. Ebenso können monofunktionelle Polysiloxane zur Oberflächenmodifizierung der Partikel eingesetzt werden, beispielsweise mit Trimethylsilylgruppen endverkappte α-Halogen-, α-Alkoxy- und α-Hydroxydimethylpolysiloxane.

Die erfindungsgemäßen Silicon(meth-)acrylat-Partikel zeichnen sich dadurch aus, dass sie durch das erfindungsgemäße Verfahren erhältlich sind und somit ein Polymer aufweisen, welches durch Polymerisation von Siloxan(meth-)acrylat/-en der Formel (I) und ggf. anderen Monomeren ggf. in Gegenwart weiterer Komponenten wie beispielsweise Füllstoffen, Hilfs- oder Aktivstoffen etc. in Gegenwart eines Radikalstarters, insbesondere eines Redoxsystems als Radikalstarter erhalten wurde. Bevorzugte erfindungsgemäße Silicon(meth-)acrylat-Partikel sind solche, die eine Kern-Hülle-Struktur besitzen (so genannte Silicon(meth-)acrylat-Komposit-Partikel). Bei diesen umschließt eine Hülle, wobei die Hülle vorzugsweise durch partikuläre Emulgatoren gebildet wird, den inneren Kern, der das polymerisierte Silicon(meth-)acrylat aufweist. Besonders bevorzugte Silicon(meth-)acrylat-Partikel sind solche, bei denen die Hülle aus den oben genannten anorganischen Partikeln gebildet wird, deren Oberfläche vorzugsweise modifiziert ist.

Bevorzugte Silicon(meth-)acrylat-Komposit-Partikel können solche sein, bei denen die Hülle modifiziert ist. Eine solche Modifizierung kann z. B. mit kationischen Substanzen, wie organischen Ammonium-Ionen oder kationischen Polymeren, kationischen Siloxanen, organischen Polymeren, wie beispielsweise Polyacrylaten, Carbonsäuren oder Carbonsäureanionen, Chelatbildnern, Diketonen, Siloxanen oder kondensierten Silanen wie oben beschrieben erfolgt sein. Dabei kann die Oberflächenmodifizierung physikalisch oder chemisch an das Polymerpartikel gebunden sein. Weiterhin können die Oberflächenmodifikatoren funktionelle Gruppen tragen, wie beispielsweise bei der Verwendung funktioneller Silane. Die Oberflächenmodifikatoren können aus diskreten Molekülen bestehen, aber auch quervernetzt sein.

Neben dem Silicon(meth-)acrylat können die Partikel aus ggf. bei der Polymerisation eingesetzten Comonomeren entstandene Komponenten aufweisen. Diese Comonomeren können vollständig oder teilweise in das Polysiloxan-(meth)acrylat-Netzwerk einreagiert sein oder auch als eigenständiges Netzwerk vorliegen. Ebenso sind Mischformen dieser beschriebenen Grenzfälle möglich und Teil dieser Erfindung.

Es kann vorteilhaft sein, wenn die Partikel weitere Komponenten aufweisen, die keine Bestandteile sind, die aus den Emulgatoren, Monomeren oder Comonomeren hervorgegangen sind. Solche Komponenten können funktionelle Komponenten, wie z. B. UV-Schutz-Pigmente, oder nicht funktionelle Komponenten, wie z. B. Füllstoffe, sein. Der Gehalt an solchen weiteren Komponenten kann von 0,01 bis 99 Gew.-%, bevorzugt von 0,1 bis 80 Gew.-% und besonders bevorzugt von 1 bis 50 Gew.-% bezogen auf den Gehalt an Silicon(meth-)acrylat betragen. Die weiteren Komponenten können nachträglich den bereits polymerisierten Partikeln durch Quellung und Diffusion zugesetzt werden. Dies kann auch unter Zuhilfenahme eines Lösemittels geschehen, welches nachher wieder entfernt wird. Es ist aber auch möglich, die weiteren Komponenten beim Herstellprozess zuzusetzen (siehe oben). Insbesondere können die weiteren Komponenten in Schritt a) des erfindungsgemäßen Verfahrens der organischen Phase zugesetzt werden. Die weiteren Komponenten können dabei gelöst in der Polymermatrix oder auch durch eine ggf. labile kovalente Bindung an die Matrix angebunden vorliegen.

Als weitere Komponenten können in den erfindungsgemäßen Partikeln z. B. Farbstoffe, Geruchsstoffe, Weichmacher, Pheromone, Hormone, Wuchsstoffe, kosmetische Öle und Wirkstoffe, Desinfektionsmittel, antimikrobielle Wirkstoffe, UV-Absorber, Antioxidantien, Biozide, Konservierungsmittel, pharmazeutische Wirkstoffe u. a. m. vorhanden sein.

Als weitere Komponenten können in den erfindungsgemäßen Partikeln insbesondere lineare oder verzeigte, mit Trimethylsiloxy-Gruppen endverkappte Dimethylpolysiloxane, cyclische Dimethylpolysiloxane, cyclische Methylphenylsiloxane, mit Trimethylsiloxy-Gruppen endverkappte Methylphenylpolysiloxane, mit Trimethylsiloxy-Gruppen endverkappte Dimethylsiloxan-Methylphenylsiloxan-Copolymere, mit Trimethylsiloxy-Gruppen endverkappte Dimethylsiloxan-methylfluoroalkylsiloxan-Copolymere, dabei ist beispielsweise der Fluoroalkylrest eine 3,3,4,4,5,5,6,6,7,7,8,8,8-Tridecafluorooctyl- oder eine 3,3,3-Trifluoropropylgruppe, α,ω-Dihydroxypolydi-methylsiloxane, end- und/oder kammständig alkoxylierte Polydimethylsiloxane, end- und/oder kammständige alkylierte Polydimethylsiloxane, sowie Mischformen aus end- und/oder kammständigen alkylierten und alkoxylierten Polydimethylsiloxanen, quaternäre (Organo-)Polysiloxane, wie ABIL® Quat 3272 und ABIL® Quat 3474 (auch Quaternium-80 genannt), Alkane, wie beispielsweise Hexan und höhere Homologe oder Cycloalkane und höhere Homologe sowie flüssige Paraffine und Isoparaffine, Squalan, aromatische Kohlenwasserstoffe wie beispielsweise Benzol oder Toluol, halogenierte Kohlenwasserstoffe wie beispielsweise Kohlenstofftetrachlorid oder Methylenchlorid, Ketone wie beispielsweise Aceton, Diethylketon oder Methylisobutylketon, Alkohole wie beispielsweise Undecylalkohol, Stearylalkohol, Cetylstearylalkohol, Oleylalkohol, Ether wie beispielsweise Dibutylether, Ester wie beispielsweise Bis(2-ethylhexyl)carbonat, Isononylisononanoat, Isopropyllaurat, Isopropylpalmitat, Hexyllaurat, Isopropylmyristat, Myristylmyristat, Cetylmyristat, 2-Octyldecylmyristat, Isopropylpalmitat, 2-Ethylhexylpalmitat, Butylstearat, Decyloleat, 2-Octyldodecyloleat, Myristyllactat, Cetyllactat, Lanolinacetat, natürliche oder naturidentische Öle wie beispielsweise Avocadoöl, Mandelöl, Olivenöl, Kakaoöl, Jojobaöl, Sesamöl, Sonnenblumenöl, Sojaöl, Kamelienöl, Zedernöl, Aprikosenkernöl, Rizinusöl, Nerzöl, Murmeltierfett, Baumwollsamenöl, Kokosöl, Eiöl, Schweineschmalz, Glykolester wie beispielsweise Polypropylenglykolmonooleat oder Neopentylglykol-2-ethylhexanoat, Glycerinester wie beispielsweise Gylcerintriisostearat oder Glycerinester der Kokosfettsäure sowie alkoxylierte Fettalkohole wie beispielsweise Laurylalkoholethoxylate oder Cetylalkoholpropoxylate, Terpenalkohole, wie z. B. Citronellol, Menthol, Linalool, Farnesol, Nerolidol, Nerol, Geraniol, Borneol, Ipsenol, Bisabolol oder Terpineol, Terpene wie beispielsweise Menthan, Terpinen, Phellandren, Pinen oder Limonen, Terpenaldehyde, wie beispielsweise Citral, Terpenketone, wie beispielsweise Menthon, Pulegon oder Carvon, Terpenderivate, wie beispielsweise Campher, Diterpene, wie beispielsweise Retinol, phenolische Substanzen, wie beispielsweise Thymol, Eugenol, Tocopherol oder Vanillin, Pheromone, wie beispielsweise Verbenon, Cholesterol-Derivate wie beispielsweise Testosteron, Androsteron, Östradiol oder Cortison, Antibiotika wie beispielsweise Metronidazol oder Dexamethason, Fungizide wie beispielsweise Orthophenylphenol oder Thiabendazol, Antimykotika wie beispielsweise Ketoconazol oder Tolnaftat sowie andere mehr vorhanden sein.

Besonders bevorzugte weitere Komponenten für Kosmetikanwendungen sind dabei Tocopherol, Tocopherolacetat, Tocopherolpalmitat, Ascorbinsäure, Ascorbylpalmitat, Desoxyribonucleinsäure, Coenzym Q10, Retinol- und Retinylderivate, Bisabolol, Allantoin, Phytantriol, Panthenol, AHA-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe bevorzugt. Auch Dehydroxyaceton und organische Sonnenschutzfilter können als weitere Komponenten enthalten sein. Die vorgenannten weiteren Komponenten können insbesondere als funktionelle Komponenten in den erfindungsgemäßen Partikeln vorhanden sein. Handelt es sich bei den funktionellen Komponenten um Radikalfänger, so werden solche funktionelle Komponenten erst nach Abschluss des radikalischen Polymerisationsschrittes b) den Partikeln zugefügt. Dies kann z. B. durch Quellung/Diffusion erfolgen.

Die erfindungsgemäßen Silicon(meth-)acrylat-Partikel können eine oder mehrere Substanzen, insbesondere ausgewählt aus den oben genannten weiteren Komponenten beinhalten, welche aus den Partikeln freigesetzt werden können. Die Freisetzung kann dabei in den entsprechenden Anwendungen über einen längeren Zeitraum hinweg erfolgen. Die Freisetzung kann z. B. durch Diffusion oder Hydrolyse-Reaktionen und nachfolgender Diffusion erfolgen.

Als freizusetzende Substanzen können beispielsweise kosmetische Öle und Wirkstoffe, Duftstoffe, pharmazeutische Wirkstoffe, antimikrobielle Wirkstoffe, auch zum Beispiel Silber und silberhaltige Verbindungen, sowie Farb- und Konservierungsstoffe in den Partikeln vorhanden sein. Diese Substanzen können sowohl gelöst als auch eingebettet in der Silicon(meth-)acrylatmatrix als auch durch eine labile chemische Bindung an die Silicon(meth-)acry-latmatrix gebunden vorliegen. Die freizusetzenden Substanzen können insbesondere die oben genannten weiteren Komponenten sein.

Die erfindungsgemäßen bzw. erfindungsgemäß hergestellten Silicon(meth-)acrylatpartikel können allein oder in Mischung mit weiteren Partikeln, Pigmenten und/oder weiteren üblichen Zusatzstoffen in Form von Pulvern oder Dispersionen in Beschichtungs-, Kleb- oder Dichtstoffen, in Polymeren, in Entschäumern, in Netz- und Verlaufshilfsmitteln, in kosmetischen oder pharmazeutischen Zubereitungen sowie Pflegemitteln, in Putz- und Reinigungsmitteln oder in Anwendungen zur Veränderung der Grenzflächeneigenschaften von festen und flüssigen Substraten, wie z. b. den haptischen Eigenschaften, Hydrophobierung oder Veränderung von Gleit- und/oder Trenneigenschaften, verwendet werden.

Erfindungsgemäße Zusammensetzungen enthalten die erfindungsgemäßen bzw. die erfindungsgemäß hergestellten Silicon(meth-)acrylat-Partikel. Solche erfindungsgemäßen Zusammensetzungen können z. B. Dispersionen von Silicon(meth-)acrylat-Komposit-Partikeln in wässrigen oder organischen Medien sein, wobei der Dispersion ggf. ein Dispergierhilfsmittel, ein Tensid und/oder ein Verdicker zugesetzt sein kann.

Zur Herstellung von solchen Dispersionen werden die erfindungsgemäß hergestellten Partikel in einem Medium, beispielsweise Wasser, Alkoholen, aliphatischen oder aromatischen Kohlenwasserstoffen sowie Siliconen, dispergiert. Dabei können die Partikel im umgebenden Medium elektrostatisch, z. B. über den pH-Wert, sterisch, z. B. mittels Dispergieradditiven bzw. Emulgatoren, oder auch elektrosterisch stabilisiert werden. Es können anionische, kationische, amphotere oder nichtionische Tenside bzw. Mischungen der vorgenannten Substanzklassen bei der Herstellung der Dispersionen zum Einsatz kommen. Kationische oberflächenaktive Verbindungen können beispielsweise ausgewählt werden aus Salzen primärer, sekundärer oder tertiärer Amine, Alkyltrimethylammoniumsalzen, Dialkyldimethylammoniumsalzen, Trialkylmethylammoniumsalzen, Tetraalkylammoniumsalzen, alkoxylierten Alkylammoniumsalzen, Alkylpyridiniumsalzen oder N,N-Dialkylmorpholiniumsalzen. Anionische oberflächenaktive Verbindungen können beispielsweise ausgewählt werden aus Salzen aliphatischer Carbonsäuren, Alkylbenzolsulfonaten, Alkylnaphthylsulfonaten, Alkylsulfonaten, Dialkylsulfosuccinaten, α-Olefinsulfonaten, Salzen α-sulfonierter aliphatischer Carbonsäuren, N-Acyl-N-Methyltauraten, Alkysulfaten, sulfatierten Ölen, polyethoxylierten Alkylethersulfaten, polyethoxylierten Alkylphenylethersulfaten, Alpkylphosphaten, polyethoxylierten Alkylethersulfaten, polyethoxylierten Alkylphenylethersulfaten und Kondensaten aus Formaldehyd und Naphthylsulfonaten. Amphotere oberflächenaktive Verbindungen können beispielsweise ausgewählt werden aus N,N-Dimethyl-N-alkyl-N-carboxymethylammoniumbetainen, N,N-Dialkylaminoalkylencarboxylaten, N,N,N-Trialkyl-N-sulfo-alkylenammoniumbetainen, N,N-Dialkyl-N,N-bispolyoxy-ethylenammoniumsulfatesterbetainen, 2-Alkyl-1-carboxymethyl-1-hydroxyethylimidazoliniumbetainen.

Nichtionische oberflächenaktive Verbindungen können beispielsweise ausgewählt werden aus polyethoxylierten Alkylethern, polyethoxylierten Alkenylethern, polyethoxylierten Alkylphenylethern, polyethoxylierten Polystyrolphenylethern, Polyoxyethylen-Polyoxypropylenglykolen, Polyoxyethylen-Polyoxypropylenalkylethern, partiellen Estern aliphatischer Carbonsäuren mit polyfunktionellen Alkoholen, beispielsweise Sorbitanester, aliphatischen Glycerinestern, aliphatischen Polyglycerinestern, aliphatischen Decaglycerinestern, (gemischten) aliphatischen Estern von Ethylenglykol/Pentaerythrit, (gemischten) aliphatischen Estern von Propylenglykol/Pentaerythrit, polyethoxylierten aliphatischen Partialestern polyfunktioneller Alkohole, beispielsweise polyethoxylierten aliphatischen Sorbitanpartialestern, ethoxylierten aliphatischen Glycerinestern, gemischt ethoxylierten/aliphatisch veresterten Säuren, aliphatischen Carbonsäureestern von Polyglycerinen, polyethoxyliertem Rizinusöl, Diethanolamiden aliphatischer Carbonsäuren, polyethoxylierten Alkylaminen, aliphatischen Partialestern des Triethanolamins, Trialkylaminoxiden sowie polyalkoxylierten Organopolysiloxanen. Solche Dispergieradditive können beispielsweise ausgewählt werden aus dem Produktportfolio der Evonik Goldschmidt GmbH, die dort beispielsweise unter den Namen "Tego® Dispers" oder "Tegopren®" erhältlich sind. Der Gehalt an solchen oberflächenaktiven Substanzen kann zwischen 0,1 und 50 Gew.-%, vorzugsweise zwischen 1 und 30 Gew.-%, bezogen auf die Dispersion betragen. Der Gehalt an dispergierten Partikeln in der Dispersion liegt vorzugsweise bei 0,1 bis 80 Gew.-%, bevorzugt bei 1 bis 40 Gew.-%.

Zur Stabilisierung und Einstellung der gewünschten Viskosität können den Dispersionen auch weitere Stoffe zugesetzt werden. Dazu zählen beispielsweise mit dem Dispergiermedium mischbare Lösemittel oder auch lösliche Polymere, beispielsweise Xanthangummi, Guarmehl, Carboxymethylzellulose, Polyvinylakohol, Polyvinylpyrrolidon, Carboxyvinylpolymere, Polyacrylate, Hydroxyethylzellulose, Polyethylenimine, polyethoxyliertes Glykolstearat sowie auch Tone, Schichtsilikate, pyrogene Oxide, wie AEROSIL@ (Evonik Degussa GmbH), Hydroxyfettsäureglyceride, Hydroxyfettsäuren, Aluminiumtristearat, Polyolefinwachse und Amidwachse.
Ebenso können den Dispersionen weitere funktionelle Stoffe zugesetzt werden, dazu zählen beispielsweise filmbildende Poly(meth-)acrylate, Silicon/(Meth-)AcrylatCopolymere, Poly-N-Acylalkylenimine, Poly-N-Methylpyrrolidone, Siliconharze mit fluorierten organischen Gruppen, Amino- oder Silanolgruppen, Antioxidantien wie beispielsweise BHA, BHT, Ascorbinsäure und γ-Orizanol, Frostschutzmittel wie beispielsweise Ethanol, Ethylenglykol, 1,3-Butylenglykol, Proylenglykol, Glycerin oder Isopropanol, antimikrobielle und konservierende Stoffe wie Triclosan und Triclocarban und Hexachlorophen, Komplexbildner wie beispielsweise EDTA (Säure und Salze), Zitronensäure und Etidronsäure sowie deren Salze, UV-Absorber wie beispielsweise Derivate des Benzophenons, des Benzotriazols, Zimtsäureester oder partikuläre UV-Absorber wie beispielsweise ZnO oder TiO₂, Farbstoff und Färbemittel, Pigmente, Sprühhilfsmittel, Netzmittel, Vitamine, Wuchsstoffe, Hormone sowie Duftstoffe.

Die Herstellung der Dispersionen mit den erfindungsgemäßen Partikel kann mittels der üblichen Methoden entsprechend dem Stand der Technik erfolgen, jedoch ist es vorteilhaft, die nach der Polymerisation gemäß Schritt b) des erfindungsgemäßen Verfahrens und Waschen mit Alkohol/-en oder Wasser entstandenen Partikel ohne vorheriges Trocknen zu beispielsweise wässrigen Dispersionen weiter zu verarbeiten. Dies ist auch möglich, wenn beispielsweise eine gewünschte Oberflächenmodifikation direkt aus wässriger oder alkoholischer Phase erfolgen kann, was sich günstig auf die Verfahrensökonomie auswirkt.

Die erfindungsgemäßen Silicon(meth-)acrylat-Partikel bzw. diese enthaltende Dispersionen können Verwendung finden als Zusatzstoffe in Kosmetika und Toilettenartikeln, z. B. als Mattierungsmittel, als Absorber für Hautfette oder zur Erzeugung eines seidigen Hautgefühls, als Additive zur Verbesserung der mechanischen Eigenschaften von Polymeren und Lacken oder Beschichtungen, z. B. zur Erhöhung der Abrieb- oder Kratzfestigkeit, der Flexibilität und auch der Schlagzähigkeit, weiterhin als Antiblockiermittel zur Verbesserung der Gleiteigenschaften verschiedenster Oberflächen, als Fließ- oder Dispergierhilfsmittel in Pulvern, als Additive in Tonern, als mildes Abrasivum in Wasch- und Pflegeformulierungen sowie als über einen längeren Zeitraum hinweg Wirkstoffe oder Hilfsstoffe freisetzende Formulierungbestandteile bzw. Trägermaterialien.

Die erfindungsgemäßen Zusammensetzungen können insbesondere auch ein Beschichtungs-, Kleb- oder Dichtstoff, ein Polymer, ein Entschäumer, Netz- und/oder Verlaufshilfsmittel, ein Kosmetikum, ein Pflegemittel, ein Medizinprodukt, ein Pharmazeutikum, ein Waschmittel, ein Putz- und/oder Reinigungsmittel, ein Hydrophobierungsmittel, ein Gleitmittel oder ein Trennmittel sein.

### Beispiele:

### Beispiel 1:

### Partikel aus Siliconacrylat TEGO® RC 726

7.600 g demineralisiertes Wasser und 276 g Ludox SM-AS wurden vermischt und mit Salzsäure auf pH 7 eingestellt. Das Gemisch wurde im Rührkessel eines Co-Twister Homogenizers (Symex) vorgelegt und unter Rühren wurden 2.156 g TEGO® RC 726 (Evonik Goldschmidt GmbH) zugefügt. Nach dem Schließen des Kessels wurde unter langsamem Rühren der Kessel bis auf 50 mbar evakuiert und nach Abklingen der Schaumentwicklung wieder auf 800 mbar belüftet. Das Gemisch wurde anschließend im Anlagenkreislauf bei einer Rotorumdrehungszahl von 2.000 Upm und einer Differenzgeschwindigkeit von 20 m/s für 15 min voremulgiert. Nach Einsaugen von 64,3 g einer 5 Gew.-%igen, wässrigen CTAB-Lösung (Cetyltrimethylammoniumbromid) sowie anschließend 420 g demineralisierten Wassers wurde für weitere 45 min bei gleichen Bedingungen emulgiert. Die erhaltene Voremulsion wurde durch Durchleiten durch einen Homogenisator (in den Beispielen wurde jeweils ein Mikrofluidizer der Firma Microfluidics eingesetzt.) mit einer Interaktionskammer mit 200 µm Durchmesser bei 800 bar Druck homogenisiert.

Zur Polymerisation wurden 850 g der Emulsion in einem 2 l Rundkolben mit einer Lösung aus 6,8 g Ammoniumperoxodisulfat in 20 ml demineralisiertem Wasser versetzt. Danach wurde unter Rühren 45 min lang ein Stickstoffstrom eingeleitet. Anschließend wurde eine Lösung von 27,2 g Dinatriumhydrogenphosphat in 100 ml demineralisiertem Wasser sowie 4,25 g einer wässrigen 38 Gew.-%igen Natriumhydrogensulfitlösung unter Stickstoff zugegeben. Die Reaktionsmischung wurde weitere 2 h gerührt und anschließend über Nacht stehen gelassen. Die erhaltenen Partikel wurden abgenutscht, durch Aufschlämmen mit Wasser und Ethanol gewaschen und in einem Vakuumtrockenschrank bei 50 °C bis zur Massenkonstanz getrocknet.

### Beispiel 2:

### Partikel aus Siliconacrylat TEGO® RC 902

4.650 g demineralisiertes Wasser und 160 g Ludox SM-AS wurden vermischt und mit Salzsäure auf pH 7 eingestellt. Das Gemisch wurde im Rührkessel eines Co-Twister Homogenizers (Symex) vorgelegt und unter Rühren wurden 1.250 g TEGO® RC 902 (Evonik Goldschmidt-GmbH) zugefügt. Nach dem Schließen des Kessels wurde unter langsamem Rühren der Kessel bis auf 50 mbar evakuiert und nach Abklingen der Schaumentwicklung wieder auf 800 mbar belüftet. Das Gemisch wurde anschließend im Anlagenkreislauf bei einer Rotorumdrehungszahl von 2.000 Upm und einer Differenzgeschwindigkeit von 20 m/s für 15 min voremulgiert. Nach Einsaugen von 37,3 g einer wässrigen 5 Gew.-%igen CTAB-Lösung (Cetyltrimethylammoniumbromid) sowie anschließend 245 g demineralisiertes Wassers wurde für weitere 45 min bei gleichen Bedingungen emulgiert. Die erhaltene Voremulsion wurde durch Durchleiten durch einen Homogenisator mit einer Interaktionskammer mit 200 µm Durchmesser bei 800 bar Druck homogenisiert.

Zur Polymerisation wurden 850 g der Emulsion in einem 21 Rundkolben mit einer Lösung aus 6,8 g Ammoniumperoxodisulfat in 20 ml demineralisiertem Wasser versetzt. Danach wurde unter Rühren 45 min lang ein starker Stickstoffstrom eingeleitet. Anschließend wurde eine Lösung von 27,2 g Dinatriumhydrogenphosphat in 100 ml demineralisiertem Wasser sowie 4,25 g einer wässrigen 38 Gew.-%igen Natriumhydrogensulfitlösung unter Stickstoff zugegeben. Die Reaktionsmischung wurde weitere 2 h gerührt und anschließend über Nacht stehen gelassen. Die erhaltenen Partikel wurden abgenutscht, durch Aufschlämmen mit Wasser und Ethanol gewaschen und in einem Vakuumtrockenschrank bei 50 °C bis zur Massenkonstanz getrocknet.

### Beispiel 3:

### Partikel aus Siliconacrylat TEGO® RC 2015

4.000 g demineralisiertes Wasser und 800 g Ludox SM-AS wurden vermischt und mit Salzsäure auf pH 7 eingestellt. Das Gemisch wurde zusammen mit 18.600 g demineralisiertem Wasser im Rührkessel eines Co-Twister Homogenizers (Symex) vorgelegt und unter Rühren wurden 6.250 g TEGO® RC 2015 (Evonik Goldschmidt GmbH) zugefügt. Nach dem Schließen des Kessels wurde unter langsamem Rühren der Kessel bis auf 50 mbar evakuiert und nach Abklingen der Schaumentwicklung wieder auf 600 mbar belüftet. Das Gemisch wurde anschließend im Anlagenkreislauf bei einer Rotorumdrehungszahl von 3.500 Upm und einer Differenzgeschwindigkeit von 40 m/s für 45 min voremulgiert. Nach Einsaugen von 157,5 g einer wässrigen, 5 Gew.-%igen CTAB-Lösung (Cetyltrimethylammoniumbromid) sowie anschließend von 400 g demineralisiertem Wasser wurde für weitere 45 min bei gleichen Bedingungen emulgiert. Vor dem Ablassen des Gemisches wurde dieses bei einer Rotorumdrehungszahl von 2.000 Upm mit gleichlaufenden Rotoren bei 200 mbar entlüftet. Die erhaltene Voremulsion wurde durch Durchleiten durch einen Homogenisator mit einer Interaktionskammer mit 200 µm Durchmesser bei 600 bar Druck homogenisiert.

Zur Polymerisation wurden 850 g der Emulsion in einem 2 1 Rundkolben mit einer Lösung aus 6,8 g Ammoniumperoxodisulfat in 20 ml demineralisiertem Wasser versetzt. Danach wurde unter Rühren 45 min lang ein starker Stickstoffstrom eingeleitet. Anschließend wurde eine Lösung von 27,2 g Dinatriumhydrogenphosphat in 100 ml demineralisiertem Wasser sowie 4,25 g einer wässrigen 38 Gew.-%igen Natriumhydrogensulfitlösung unter Stickstoff zugegeben. Die Reaktionsmischung wurde weitere 2 h gerührt und anschließend über Nacht stehen gelassen. Die erhaltenen Partikel wurden abgenutscht, durch Aufschlämmen mit Wasser und Ethanol gewaschen und in einem Vakuumtrockenschrank bei 50 °C bis zur Massenkonstanz getrocknet.

### Beispiel 4:

### Partikel aus einer Mischung von Siliconacrylaten TEGO® RC 726 und TEGO® RC 902

186 g demineralisiertes Wasser wurden mit 12,8 g Ludox SM-AS vermischt und mit verdünnter HCl auf pH 7 eingestellt. 25 g RC726 und 25 g TEGO® RC 902 (Evonik Goldschmidt GmbH) wurden zu einer Lösung vermischt und mit der wässrigen Phase 15 Minuten lang in einem VakuumDissolver mit Mizerscheibe bei 4.000 Upm voremulgiert. Anschließend wurden 2,5 g einer 5 Gewichts-%igen, wässrigen CTAB-Lösung zugefügt und für weitere 30 Minuten bei 4.000 Upm im Vakuumdissolver emulgiert.

Die erhaltene Voremulsion wurde durch Durchleiten durch einen Homogenisator mit einer Interaktionskammer mit einem Mikrokanal mit 200 µm Durchmesser bei 800 bar Druck homogenisiert.

Zur Polymerisation wurden 100 g der erhaltenen Emulsion mit 0,8 g Ammoniumperoxodisulfat in 5 ml demin. Wasser versetzt und unter Rühren über 30 Minuten mit einem kräftigen Stickstoffstrom gespült. Anschließend wurde eine Lösung aus 3,2 g Dinatriumhydrogenphosphat, 0,5 g einer 38 Gewichts-%igen, wässrigen Natriumhydrogensulfitlösung und 30 g demin. Wasser zugefügt und weitere 2 Stunden unter Stickstoff gerührt. Die erhaltene Dispersion wurde über Nacht stehen gelassen. Die erhaltenen Partikel wurden abgenutscht, durch Aufschlämmen mit Wasser und Ethanol gewaschen und in einem Vakuumtrockenschrank bei 50 °C bis zur Massenkonstanz getrocknet.

### Beispiel 5:

### Partikel aus Siliconacrylat TEGO® RC 726 und Methylmethacrylat (MMA)

188 g demin. Wasser wurden mit 12,6 g Ludox SM-AS vermischt und mit verd. Salzsäure auf pH 7 eingestellt. Dazu wurde eine Lösung aus 45 g TEGO® RC726 (Evonik Goldschmidt GmbH) und 5 g MMA gegeben und für 15 min in einem Vakuumdissolver mit Mizerscheibe bei 4.000 Upm voremulgiert. Anschließend wurden 2,6 g einer 5 Gewichts-%igen wässrigen CTAB-Lösung hinzugefügt und das Gemisch für weitere 30 Minuten im Vakuumdissolver bei 4.000 Upm emulgiert.

Die erhaltene Voremulsion wurde durch Durchleiten durch einen Homogenisator mit einer Interaktionskammer mit einem Mikrokanal mit 200 µm Durchmesser bei 800 bar Druck homogenisiert.

Zur Polymerisation wurden 100 ml der erhaltenen Emulsion mit 0,8 g Ammoniumperoxodisulfat in 5 ml demin. Wasser versetzt und unter Rühren für 30 Minuten mit einem kräftigen Stickstoffstrom gespült. Anschließend wurde eine Lösung aus 3,2 g Dinatriumhydrogenphosphat, 0,5 g einer 38 Gewichts-%igen, wässrigen Natriumhydrogensulfitlösung und 30 g demin. Wasser zugefügt und weitere 2 Stunden unter Stickstoff gerührt. Die erhaltene Dispersion wurde über Nacht stehen gelassen. Die erhaltenen Partikel wurden abgenutscht, durch Aufschlämmen mit Wasser und Ethanol gewaschen und in einem Vakuumtrockenschrank bei 50 °C bis zur Gewichtskonstanz getrocknet.

### Beispiel 6:

### Partikel aus Siliconacrylat TEGO® RC 726 gefüllt mit AEROSIL@ R 974

In 90 g TEGO® RC 726 (Evonik Goldschmidt GmbH) wurden in einem Vakuumdissolver 10 g AEROSIL@ R974 (Evonik Degussa GmbH) bei 4000 Upm für 10 min eindispergiert. 186 g demineralisrtes Wasser wurden mit 12,8 g Ludox SM-AS vermischt und mit verd. HCl auf pH 7 eingestellt. Dazu wurden 50 g der hergestellten Dispersion von AEROSIL@ R 974 in Siliconacrylat TEGO® RC 726 gegeben und für 15 min in einem Vakuumdissolver mit Mizerscheibe bei 4.000 Upm voremulgiert. Anschließend wurden 2,6 g einer 5 Gewichts-%igen wässrigen CTAB-Lösung hinzugefügt und das Gemisch für weitere 30 Minuten im Vakuumdissolver bei 4.000 Upm emulgiert.

Die erhaltene Voremulsion wurde durch Durchleiten durch einen Homogenisator mit einer Interaktionskammer mit einem Mikrokanal mit 200 µm Durchmesser bei 800 bar Druck homogenisiert.

Zur Polymerisation wurden 100 ml der erhaltenen Emulsion mit 0,8 g Ammoniumperoxodisulfat in 5 ml demin. Wasser versetzt und unter Rühren für 30 Minuten mit einem kräftigen Stickstoffstrom gespült. Anschließend wurde eine Lösung aus 3,2 g Dinatriumhydrogenphosphat, 0,5 g einer 38 Gewichts-%igen, wässrigen Natriumhydrogensulfitlösung und 30 g demin. Wasser zugefügt und weitere 2 Stunden unter Stickstoff gerührt. Die erhaltene Dispersion wurde über Nacht stehen gelassen. Die erhaltenen Partikel wurden abgenutscht, durch Aufschlämmen mit Wasser und Ethanol gewaschen und in einem Vakuumtrockenschrank bei 50 °C bis zur Gewichtskonstanz getrocknet.

### Beispiel 7:

### Partikel aus Siliconacrylat TEGO® RC 726 mit Menthol

90 g Siliconacrylat TEGO® RC 726 (Evonik Goldschmidt GmbH) wurden mit einer Lösung von 10 g Menthol in 20 g Aceton vermischt und das Aceton im Vakuum abgezogen. 186 g demin. Wasser wurden mit 12,8 g Ludox SM-AS vermischt und mit verd. HCl auf pH 7 eingestellt. Dazu wurden 50 g der Menthol-Lösung gegeben und für 15 min in einem Vakuumdissolver mit Mizerscheibe bei 4.000 Upm voremulgiert. Anschließend wurden 2,6 g einer 5 Gewichts-%igen wässrigen CTAB-Lösung hinzugefügt und das Gemisch für weitere 30 Minuten im Vakuumdissolver bei 4.000 Upm emulgiert. Die erhaltene Voremulsion wurde durch Durchleiten durch einen Homogenisator mit einer Interaktionskammer mit Mikrokanal mit 200 µm Durchmesser bei 800 bar Druck homogenisiert.

Zur Polymerisation wurden 100 ml der erhaltenen Emulsion mit 0,8 g Ammoniumperoxodisulfat in 5 ml demin. Wasser versetzt und unter Rühren für 30 Minuten mit einem kräftigen Stickstoffstrom gespült. Anschließend wurde eine Lösung aus 3,2 g Dinatriumhydrogenphosphat, 0,5 g einer 38 Gewichts-%igen, wässrigen Natriumhydrogensulfitlösung und 30 g demin. Wasser zugefügt und weitere 2 Stunden unter Stickstoff gerührt. Die erhaltene Dispersion wurde über Nacht stehen gelassen. Die erhaltenen Partikel wurden abgenutscht, durch Aufschlämmen zweimal mit Wasser gewaschen und bei Raumtemperatur getrocknet. Die so erhaltenen Partikel wiesen einen deutlichen Geruch nach Menthol auf.

### Beispiel 8:

### Partikel aus Siliconacrylat TEGO® RC 726 und ABIL® Quat 3474

98 g Siliconacrylat TEGO® RC 726 (Evonik Goldschmidt GmbH) wurden mit 2 g ABIL® Quat 3474 (diquaternäres Polydimethylsiloxan, Evonik Goldschmidt GmbH) vermischt. 186 g demin. Wasser wurden mit 12,8 g Ludox SM-AS vermischt und mit verd. HCl auf pH 7 eingestellt. Dazu wurden 50 g der hergestellten Lösung aus ABIL® Quat 3474 in TEGO® RC 726 gegeben und für 15 min in einem Vakuumdissolver mit Mizerscheibe bei 4.000 Upm voremulgiert. Anschließend wurden 2,6 g einer 5 Gewichts-%igen wässrigen CTAB-Lösung hinzugefügt und das Gemisch für weitere 30 Minuten im Vakuumdissolver bei 4.000 Upm emulgiert.

Die erhaltene Voremulsion wurde durch Durchleiten durch einen Homogenisator mit einer Interaktionskammer mit Mikrokanal mit 200 µm Durchmesser bei 800 bar Druck homogenisiert.

Zur Polymerisation wurden 100 ml der erhaltenen Emulsion mit 0,8 g Ammoniumperoxodisulfat in 5 ml demin. Wasser versetzt und unter Rühren für 30 Minuten mit einem kräftigen Stickstoffstrom gespült. Anschließend wurde eine Lösung aus 3,2 g Dinatriumhydrogenphosphat, 0,5 g einer 38 Gewichts-%igen, wässrigen Natriumhydrogensulfitlösung und 30 g demin. Wasser zugefügt und weitere 2 Stunden unter Stickstoff gerührt. Die erhaltene Dispersion wurde über Nacht stehen gelassen. Die erhaltenen Partikel wurden abgenutscht, durch Aufschlämmen mit Wasser und anschließend mit Ethanol gewaschen und bei 50 °C in einem Vakuumtrockenschrank getrocknet.

### Beispiel 9:

### Partikel aus Siliconacrylat TEGO® RC 902 mit Cyclomethicon (Gemisch aus Octamethylcyclotetrasiloxan und Decamethylcyclotetrasiloxan)

45 g Siliconacrylat TEGO® RC 902 (Evonik Goldschmidt GmbH) wurden mit 5 g Cyclomethicon gemischt. 186 g demineralisiertes Wasser wurden mit 3,2 g Ludox® SM-AS vermischt und mit verd. HCl auf pH 7 eingestellt. Dazu wurde die oben hergestellte Lösung von Cyclomethicon in TEGO® RC 902 gegeben und für 15 min in einem Vakuumdissolver mit Mizerscheibe bei 4.000 Upm voremulgiert. Anschließend wurden 0,65 g einer 5 Gewichts-%igen wässrigen CTAB-Lösung hinzugefügt und das Gemisch für weitere 30 Minuten im Vakuumdissolver bei 4.000 Upm emulgiert.

Die erhaltene Voremulsion wurde durch Durchleiten durch einen Homogenisator mit einer Interaktionskammer mit Mikrokanal mit 200 µm Durchmesser bei 800 bar Druck homogenisiert.

Zur Polymerisation wurden 100 ml der erhaltenen Emulsion mit 0,8 g Ammoniumperoxodisulfat in 5 ml demin. Wasser versetzt und unter Rühren für 30 Minuten mit einem kräftigen Stickstoffstrom gespült. Anschließend wurde eine Lösung aus 3,2 g Dinatriumhydrogenphosphat, 0,5 g einer 38 Gewichts-%igen, wässrigen Natriumhydrogensulfitlösung und 30 g demin. Wasser zugefügt und weitere 2 Stunden unter Stickstoff gerührt. Die erhaltene Dispersion wurde über Nacht stehen gelassen. Die erhaltenen Partikel wurden abgenutscht, durch Aufschlämmen zweimal mit Wasser gewaschen und bei Raumtemperatur getrocknet.

### Beispiel 10:

### Partikel aus Siliconacrylat TEGO® RC 902 mit Tegiloxan® 3

45 g Siliconacrylat TEGO® RC 902 (Evonik Goldschmidt GmbH) wurden mit 5 g Tegiloxan® 3 (Siliconöl 3 cSt, Evonik Goldschmidt GmbH) gemischt. 186 g demin. Wasser wurden mit 3,2 g Ludox® SM-AS vermischt und mit verd. HCl auf pH 7 eingestellt. Dazu wurde die oben hergestellte Lösung von Tegiloxan® 3 in TEGO® RC 902 gegeben und für 30 min in einem Vakuumdissolver mit Mizerscheibe bei 4.000 Upm voremulgiert. Anschließend wurden 0,65 g einer 5 Gewichts-%igen wässrigen CTAB-Lösung hinzugefügt und das Gemisch für weitere 60 Minuten im Vakuumdissolver bei 4.000 Upm emulgiert.

Die erhaltene Voremulsion wurde durch Durchleiten durch einen Homogenisator mit einer Interaktionskammer mit Mikrokanal mit 200 µm Durchmesser bei 800 bar Druck homogenisiert.

Zur Polymerisation wurden 100 ml der erhaltenen Emulsion mit 0,8 g Ammoniumperoxodisulfat in 5 ml demin. Wasser versetzt und unter Rühren für 30 Minuten mit einem kräftigen Stickstoffstrom gespült. Anschließend wurde eine Lösung aus 3,2 g Dinatriumhydrogenphosphat, 0,5 g einer 38 Gewichts-%igen, wässrigen Natriumhydrogensulfitlösung und 30 g demin. Wasser zugefügt und weitere 2 Stunden unter Stickstoff gerührt. Die erhaltene Dispersion wurde über Nacht stehen gelassen. Die erhaltenen Partikel wurden abgenutscht, durch Aufschlämmen zweimal mit Wasser gewaschen und bei Raumtemperatur getrocknet.

### Beispiel 11:

### Siliconölaufnahme von Partikeln aus Siliconacrylat TEGO® RC 902 mit Lösungsmittel Dichlormethan

10 g Siliconpartikel aus Beispiel 2 (Siliconacrylat TEGO® RC 902 polymerisiert) wurden mit einer Lösung von 2,5 g Siliconöl (Polydimethylsiloxan, 350 cSt) in 80 g Dichlormethan versetzt und über Nacht quellen gelassen. Anschließend wurde das Dichlormethan langsam im Vakuum abgezogen. Es wurde ein pulvriger, nicht klebriger Rückstand erhalten, aus dem sich durch Fingerdruck kein Siliconöl mehr auf Filtrierpapier (Schwarzband-Filter) abpressen ließ.

### Beispiel 12:

### Siliconölaufnahme von Siliconpartikeln aus Siliconacrylat TEGO® RC 726

10 g Siliconacrylat-Partikel aus Beispiel 1 wurden mit 30 g TEGILOXAN® 3 (3 cSt, Evonik Goldschmidt GmbH) versetzt und für 36 h stehen gelassen. Die gequollenen Partikel wurden über ein Schwarzbandfilter vom restlichen Öl abgesaugt, noch im Filter mit wenig Ethanol gespült und getrocknet. Es wurden 12,8 g eines trockenen, weißen Pulver erhalten, das bei starkem Andruck auf ein Filtrierpapier einen Teil der aufgenommenen Flüssigkeit wieder abgab.

### Beispiel 13:

### Siliconölaufnahme von Siliconpartikeln aus Siliconacrylat TEGO® RC 726

10 g Siliconacrylat-Partikel aus Beispiel 1 wurden mit 30 g Cyclomethicon (Gemisch aus Octamethylcyclotetrasiloxan und Decamethylcyclopentasiloxan) versetzt und für 36 h stehen gelassen. Die gequollenen Partikel wurden über ein Schwarzbandfilter vom restlichen Öl abgesaugt, noch im Filter mit wenig Ethanol gespült und getrocknet. Es wurden 14,7 g eines trockenen, weißen Pulver erhalten, das bei starkem Andruck auf ein Filtrierpapier einen Teil der aufgenommenen Flüssigkeit wieder abgab.

### Beispiel 14:

### Oberflächenmodifizierung von Partikeln aus Siliconacrylat TEGO® RC 726 mit 3-Methacryloxypropyltrimethoxysilan

9,9 g Siliconacrylat-Partikel aus Beispiel 1 wurden in 13,1 g Methanol aufgerührt, so dass eine Paste entstand.

Zu dieser Paste wurden 0,5g 3-Methacryloxypropyltrimethoxysilan (Dynasylan® MEMO, Evonik Degussa GmbH) sowie 2 Tropfen Ameisensäure gegeben und gründlich vermischt. Nach Stehen über Nacht wurden die flüchtigen Bestandteile bei 50 °C im Ölpumpenvakuum abrotiert.

### Beispiel 15:

### Partikel-Dispersion aus Siliconacrylat TEGO® RC 726 mit TEGO® CARE PL 4 und Natriumlaurylsulfat als Emulgatoren

In einem 5 l Becherglas wurden 968 g demineralisiertes Wasser mit 75 g TEGO® CARE PL 4 (nichtionischer Emulgator, Evonik Goldschmidt GmbH) und 7,5 g Natriumlaurylsulfat versetzt und unter Rühren homogenisiert. 450 g TEGO® RC 726 wurden zugefügt und für eine Stunde unter Rühren mit einer Mizer-Scheibe bei 200 Upm voremulgiert.

Die erhaltene Voremulsion wurde durch Durchleiten durch einen Homogenisator mit einer Interaktionskammer mit 200 µm Durchmesser bei 800 bar Druck homogenisiert.

1.340 g der erhaltenen Emulsion wurden in einem 4 l Vierhalskolben mit 33 g Ammoniumperoxodisulfat in 50 g demineralisiertem Wasser versetzt und unter Rühren mit einem KPG-Rührer für 30 min mit einem starken Stickstoffstrom entlüftet. Eine Lösung aus 127 g Dinatriumhydrogenphosphat-Dihydrat und 7,1 g Natriumhydrogensulfit-Lösung (38 Gewichts-%, wässrig) in 250 g warmem demineralisierten Wasser wurde zugefügt und das Reaktionsgemisch wurde unter Stickstoff für eine weitere Stunde gerührt. Nach Stehenlassen über Nacht wurde die Reaktionsmischung über ein 230 µm Schnellsieb filtriert, als Filtrat wurde eine Siliconacrylat-Partikel-Dispersion erhalten.

### Beispiele 16 bis 19, Vergleichsbeispiele V1 und V2: Anwendungsbeispiele Kosmetik

Als Vergleichsbeispiele V1 und V2 sind die entsprechenden Emulsionen ohne Siliconacrylat-Partikel dargestellt.

Bei den Beispielen 16 und 17 handelt es sich um Emulsionen vom Typ Öl-in-Wasser, Beispiel 18 repräsentiert eine Emulsion vom Typ Wasser-in-Öl.

Als Pigmente wurden in Beispiel 19 (Sonnenschutz) Titandioxid verwendet, während in Beispiel 18 (Foundation) übliche Eisenoxide in Kombination mit Titandioxid verwendet wurden. Vergleichsbeispiel V2 dient insbesondere dazu, die sensorischen Vorteile zu dokumentieren, die durch den Einsatz der erfindungsgemäßen Partikel in pigmenthaltigen Formulierungen erzielbar sind.

Bei Beispielemulsion 16 sowie bei V1 erfolgte die Emulsionsherstellung im Heiß-Heiß-Verfahren (Öl- und Wasserphase bei 70 bis 75°C nach üblichen Methoden homogenisiert) .

Die Beispielemulsion 17 zeigt, dass die erfindungsgemäßen Partikel auch problemlos in eine kaltherstellbare Emulsion eingerührt werden können. Dabei werden Öl- und Wasserphase bei Raumtemperatur kombiniert und nach üblichen Methoden homogenisiert.

Beispielemulsion 18 wurde in einem Kalt-Kaltverfahren bei Raumtemperatur hergestellt. Dabei wurde zunächst die Ölphase homogenisiert und anschließend unter schwachem Rühren langsam die Wasserphase hinzugegeben. Nach Beendigung der Wasserzugabe wurde erneut homogenisiert.

Bei Beispielemulsion 19 erfolgte die Herstellung in einem Heiß-Heiß-Prozess, indem die auf 80 ° erhitzten Öl- und Wasserphasen zusammengegeben und homogenisiert wurden.

Generell zeigen die Beispiele, dass die erfindungsgemäßen Partikel sowohl direkt in die Ölphase gegeben werden können (wie etwa in Beispiel 18), als dass sie auch nachträglich in die fertige Emulsion eingearbeitet werden können (wie in Beispiel 16 oder 17).

Die Zusammensetzungen der Beispielformulierungen und Vergleichsformulierungen werden in den nachfolgenden Tabellen 1 bis 4 angegeben.

**Tabelle 1:**

| Formulierungen und Ergebnisse von Beispiel 16 und Vergleichsbeispiel V1, Öl-in-Wasser Pflegecreme | | | |
|---|---|---|---|
| | **Beispiel** | **16** | **V1** |
| **A** | TEGO® Care 165 (Evonik Goldschmidt GmbH) (Glyceryl Stearate; PEG-100 Stearate) | 6,0 % | 6,0 % |
| | Stearyl Alcohol | 3,0 % | 3,0 % |
| | Mineral Oil | 4,0 % | 4,0 % |
| | Ethylhexyl Palmitate | 4,0 % | 4,0 % |
| **B** | Glycerin | 3,0 % | 3,0 % |
| | Wasser | 75,0 % | 80,0 % |
| **C** | Siliconacrylat-Partikel aus Bsp. 1 | 5,0 % | |
| **Z** | Konservierungsmittel, Parfum | q.a. | q.a. |
| | | | |
| | Stabilität | Gut | Gut |
| | Erscheinungsbild | Weiß, homogen | Weiß, homogen |
| | Hautgefühl | Samtigseidig, glatt; nicht stumpf | Wachsig, stumpf |

**Tabelle 2:**

| Formulireung und Ergebnisse der in Beispiel 17 kalt hergestellten Öl-in-Wasser Körperpflegelotion: | | |
|---|---|---|
| | **Beispiel** | **17** |
| **A** | TEGO® Care LTP (Evonik Goldschmidt GmbH) Sorbitan Laurate; Polyglyceryl-4 Laurate; Dilauryl Citrate) | 1,5 % |
| | Cyclopentasiloxane | 10,0 % |
| | Isohexadecane | 3,5 % |
| | Ethylhexyl Palmitate | 1,1 % |
| | TEGO® Carbomer 140 (Evonik Degussa GmbH) | 0,15 % |
| | TEGO® Carbomer 141 (Evonik Degussa GmbH) | 0,15 % |
| | Xanthan Gum | 0,1 % |
| **B** | Glycerin | 3,0 % |
| | Wasser | 79,6 % |
| **C** | NaOH (10%ige Lösung) | 0,90 % |
| **D** | Siliconacrylat-Partikel aus Bsp. 1 | 5,0 % |
| **Z** | Konservierungsmittel, Parfum | q.a. |
| | | |
| | Stabilität | Gut |
| | Erscheinungsbild | Weiß, homogen |
| | Hautgefühl | Leicht; samtig; glatt |

**Tabelle 3:**

| Wasser-in-Öl Foundation aus Beispiel 18 und Vergleichsbeispiel V2: | | | |
|---|---|---|---|
| | **Beispiel** | **18** | **V2** |
| A | ABIL® EM 90 (Evonik Goldschmidt GmbH) (Cetyl PEG/PPG-10/1 Dimethicone) | 3,0 % | 3,0 % |
| | Diethylhexyl Carbonate | 10,0 % | 10,0 % |
| | Cyclopentasiloxane | 7,6 % | 7,6 % |
| | Ethylhexyl Palmitate | 3,4 % | 3,4 % |
| | Iron Oxides | 1,8 % | 1,8 % |
| | Titanium Dioxide | 7,2 % | 7,2 % |
| | Talcum | 2,0 % | 2,0 % |
| | Siliconacrylat-Partikel aus Bsp. 1 | 2,5 % | |
| B | NaCl | 1,0 % | 1,0 % |
| | Glycerin | 2,0 % | 2,0 % |
| | Wasser | 65,5 % | 68,0 % |
| Z | Konservierungsmittel, Parfum | q.a. | q.a. |
| | | | |
| | Stabilität | Gut | Gut |
| | Erscheinungsbild | Homogen, bräunlich | Homogen, bräunlich |
| | Hautgefühl | Glatt, nicht stumpf, samtig | Etwas trocken und stumpf |

**Tabelle 4:**

| Öl-in-Wasser Sonnenschutzlotion gemäß Beispiel 19 | | |
|---|---|---|
| | **Beispiel** | **19** |
| **A** | AXOL® C 62 (Evonik Goldschmidt GmbH) (Glyceryl Stearate Citrate) | 2,0 % |
| | Cetearyl Alcohol | 1,0 % |
| | C₁₂₋₁₅ Alkyl Benzoate | 8,0 % |
| | Triisostearin | 1,0 % |
| | Diethylhexyl Carbonate | 2,75 % |
| | Tocopheryl Acetate | 0,5 % |
| | Xanthan Gum | 0,4 % |
| | Ethylhexyl Methoxy-cinnamate | 7,0 % |
| | Butyl Methoxydibenzoyl-methane | 3,0 % |
| | TEGO® Sun T 805 (Evonik Goldschmidt GmbH) (Titanium Dioxide; Trimethoxycaprylylsilane) | 2,25 % |
| | Siliconacrylat-Partikel aus Bsp. 1 | 2,5 % |
| **B** | Glycerin | 2,0 % |
| | Wasser | 67,6 % |
| **Z** | Konservierungsmittel, Parfum | q.a. |
| | | |
| | Stabilität | Gut |
| | Erscheinungsbild | Weiß, homogen |
| | Hautgefühl | Pflegend, glatt, samtig |

Die Anwendungsbeispiele zeigen, dass die erfindungsgemäßen Silicon(meth-)acrylat-Partikel in stabile kosmetische Formulierungen eingearbeitet werden können. Durch die Verwendung dieser Partikel werden die sensorischen Eigenschaften von kosmetischen Formulierungen deutlich verbessert, ohne dass sich Stabilität und das Erscheinungsbild der Beipielemulsionen verschlechtern. Insbesondere führt die Einarbeitung der Kompositpartikel zu einem samtigeren, seidigeren, weniger trockenen und weniger stumpfen Hautgefühl.

Insbesondere sind die Silicon(meth-)acrylat-Partikel auch geeignet, in Formulierungen zusammen mit Pigmenten eingesetzt zu werden, da sie das üblicherweise etwas stumpfe Hautgefühl pigmenthaltiger Formulierungen deutlich verbessern.

## Patentansprüche

1. Verfahren zur Herstellung von Silicon(meth-)acrylat-Partikeln umfassend die Schritte
a) Erzeugen einer Emulsion aus Wasser und einer organischen Phase, wobei die organische Phase, end- und/oder seitenständig mit (Meth-)AcrylatGruppen modifizierte Organopolysiloxane nach der allgemeinen Formel (I) oder deren Gemische 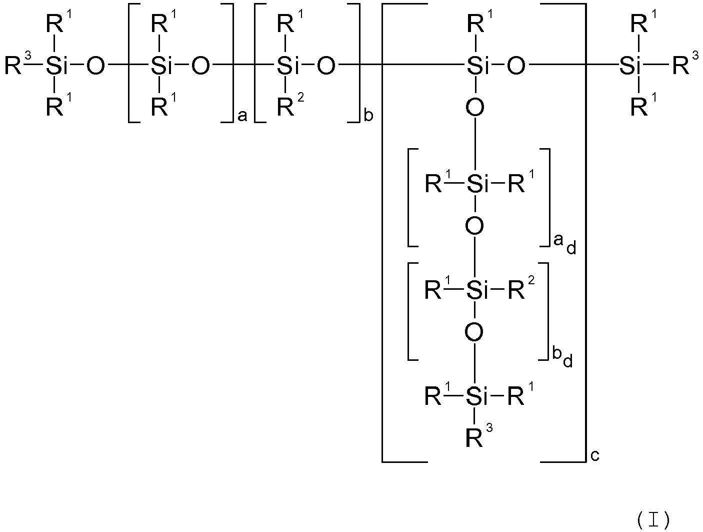 mit
R¹ = gleiche oder verschiedene Reste, ausgewählt aus linearen oder verzweigten, gesättigten, ein- oder mehrfach ungesättigten, gegebenenfalls eine oder mehrere Ether- oder Esterbrücken enthaltende lineare, cyclische oder verzweigte Alkyl-, Alkoxy-, Polyalkoxy-, Hydroxyalkyl-, Hydroxyalkoxy-, Alkenyl-, Aryl-, Aryloxy-, Hydroxyaryl-, Hydroxyaryloxy-, Alkaryl-, Alkaryloxy-, Hydroxyalkaryl-, Hydroxyalkaryloxy-, Aralkyl-, Aralkoxy-, Hydroxyaralkyl- oder Hydroxyaralkoxy-Resten mit 1 bis 20 C-Atomen,
R² = gleiche oder verschiedene, an das Si-Atom über eine Si-C-Verknüpfung oder eine Si-O-C-Verknüpfung gebundene, gegebenenfalls eine oder mehrere Ether- oder Esterbrücken enthaltende zweiwertige, optional OH-funktionalisierte Kohlenwasserstoffreste, mit 1 bis 20 C-Atomen, an welche 1 bis 5 Acrylsäure- und/oder Methacrylsäure-Einheiten und gegebenenfalls Monocarbonsäure-Einheiten mit 2 bis 10 C-Atomen, welche frei von zur Polymerisation befähigten Doppelbindungen sind, über Esterbindungen angebunden sind,
R³ = gleiche oder verschiedene Reste R¹ oder R²,
a = 0 bis 1.000,
b = 0 bis 200,
c = 0 bis 200,
a_{d} = 0 bis 1.000,
b_{d} = 0 bis 200,
wobei
der Index d für c > 0 eine ganze Zahl > 0 ist, mit der Maßgabe, dass wenn b und c = 0 sind, R³ nicht aus der gleichen Gruppe wie R¹ ausgewählt sein darf, enthält, unter Zugabe zumindest eines Emulgators und optional eines oder mehrerer Coemulgatoren,
wobei
die organische Phase die innere Phase der Emulsion bildet, und
b) Auspolymerisieren der inneren Phase in Gegenwart eines Radikalstarters, welcher der äußeren, wässrigen Phase in einer Konzentration von 0,1 bis 40 Gew.-% bezogen auf die innere Phase zugefügt wird.

2. Verfahren gemäß Anspruch 1, **dadurch gekennzeichnet, dass** in Schritt a) eine festkörperstabilisierte Emulsion erzeugt wird und als Emulgatoren partikuläre, in zumindest einer Dimension mikro- oder nanoskalige Emulgatoren, die ausgewählt sind aus der Gruppe der Metalloxide, Mischoxide, Nitride, Hydroxide, Carbonate, Silikate, Siliconharze, Silicone und/oder organischen Polymere, die zumindest (teil-)hydropho-biert sind mit mindestens einer Verbindung aus der Gruppe der Silane, Siloxane, quaternären Ammoniumverbindungen, kationischen Polymere und Fettsäuren oder deren Anionen, eingesetzt werden.

3. Verfahren nach Anspruch 1 oder 2, **dadurch gekennzeichnet, dass** mehr als 70 % der Reste R¹ in Formel (I) Methylgruppen sind.

4. Verfahren gemäß zumindest einem der Ansprüche 1 bis 3, **dadurch gekennzeichnet, dass** R² in der allgemeinen Formel(I) aus der Gruppe der Reste 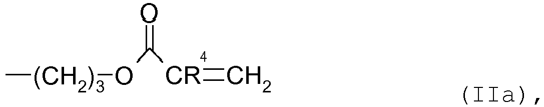 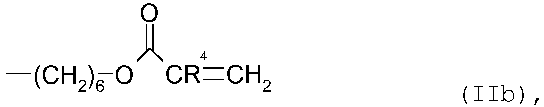 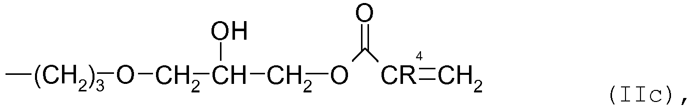 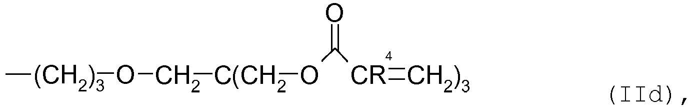 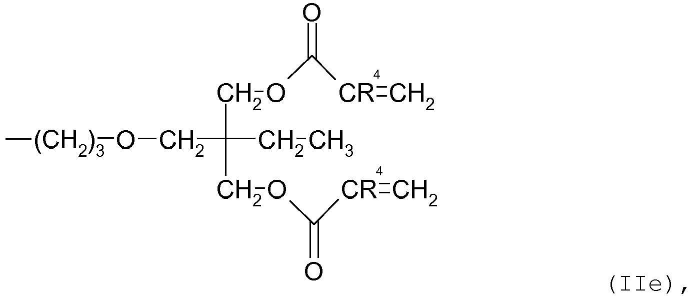 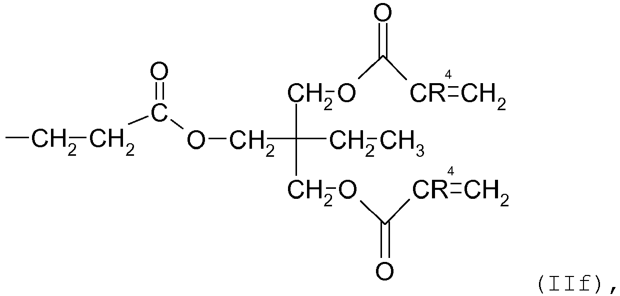 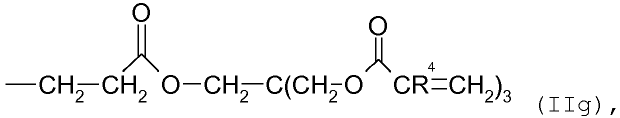 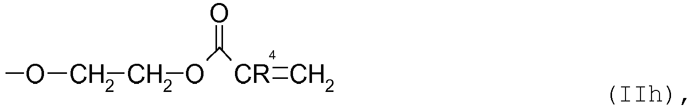 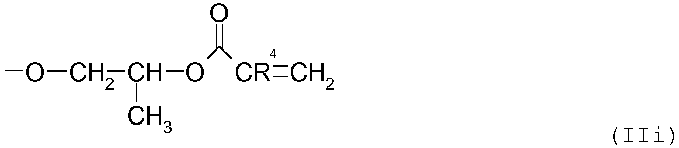 und/oder 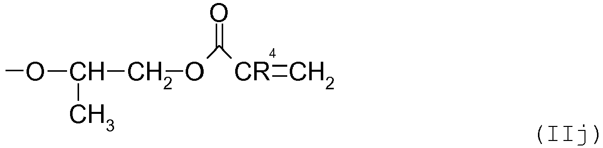 ausgewählt ist und
R⁴ Wasserstoff oder eine Methylgruppe ist.

5. Verfahren gemäß zumindest einem der Ansprüche 1 bis 4, **dadurch gekennzeichnet, dass** Silicon(meth-)acrylate der Formel (I) eingesetzt werden, bei denen a einen Wert von 0 bis 500 einnimmt.

6. Verfahren gemäß zumindest einem der Ansprüche 1 bis 5, **dadurch gekennzeichnet, dass** Silicon(meth-)acrylate der Formel (I) eingesetzt werden, bei denen b einen Wert von 0 bis 100 einnimmt.

7. Verfahren gemäß zumindest einem der Ansprüche 1 bis 6, **dadurch gekennzeichnet, dass** Silicon(meth-)acrylate der Formel (I) eingesetzt werden, bei denen c einen Wert von 0 bis 100 einnimmt.

8. Verfahren gemäß zumindest einem der Ansprüche 1 bis 7, **dadurch gekennzeichnet, dass** der organischen Phase vor Schritt b) weitere Comonomere zugefügt werden.

9. Verfahren gemäß zumindest einem der Ansprüche 1 bis 8, **dadurch gekennzeichnet, dass** der organischen Phase in Schritt a) weitere Komponenten hinzugefügt werden.

10. Verfahren gemäß zumindest einem der Ansprüche 1 bis 9, **dadurch gekennzeichnet, dass** die organische Phase Substanzen beinhaltet, welche aus den Partikeln freigesetzt werden können.

11. Verfahren gemäß zumindest einem der Ansprüche 1 bis 10, **dadurch gekennzeichnet, dass** die Emulsion in Schritt a) durch Durchleiten des Gemisches aus organischer und wässriger Phase durch und Dispergieren des Gemisches in wenigstens einer Interaktionskammer mit einer Kapillardicke von 50 bis 500 µm in einem Druckbereich von 50 bis 1.000 bar und Entspannung des Gemisches in ein Auslaß-Reservoir hergestellt wird.

12. Silicon(meth-)acrylat-Partikel erhältlich durch ein Verfahren gemäß einem der Ansprüche 1 bis 11.

13. Silicon(meth-)acrylat-Partikel gemäß Anspruch 12, **dadurch gekennzeichnet, dass** die Partikel eine Kern-Hülle-Struktur besitzen, wobei die Hülle durch partikuläre Emulgatoren gebildet wird, die einen inneren Kern aus Silicon(meth-)acrylat umschließt.

14. Silicon(meth-)acrylat-Partikel gemäß Anspruch 13, **dadurch gekennzeichnet, dass** die Hülle aus anorganischen Partikeln gebildet wird oder eine Polymerhülle ist, deren Oberfläche modifiziert ist.

15. Silicon(meth-)acrylat-Partikel gemäß zumindest einem der Ansprüche 12 bis 14, **dadurch gekennzeichnet, dass** die Partikel Substanzen beinhalten, welche aus den Partikeln freigesetzt werden.

16. Verwendung der Silicon(meth-)acrylat-Partikel gemäß zumindest einem der Ansprüche 12 bis 15 oder hergestellt gemäß zumindest einem der Ansprüche 1 bis 11 allein oder in Mischung mit weiteren Partikeln, Pigmenten und/oder weiteren üblichen Zusatzstoffen in Form von Pulvern oder Dispersionen in Beschichtungs-, Kleb- oder Dichtstoffen, in Polymeren, in Entschäumern, in Netz- und Verlaufshilfsmitteln, in kosmetischen oder pharmazeutischen Zubereitungen sowie Pflegemitteln, in Putz- und Reinigungsmitteln oder in Anwendungen zur Veränderung der Grenzflächeneigenschaften von festen und flüssigen Substraten.

17. Zusammensetzungen enthaltend Silicon(meth-)acrylat-Partikel gemäß den Ansprüchen 12 bis 15 oder hergestellt gemäß einem der Ansprüche 1 bis 11.

18. Zusammensetzung gemäß Anspruch 17, **dadurch gekennzeichnet, dass** die Zusammensetzung eine Dispersion von Silicon(meth-)acrylat-Partikeln in wässrigen oder organischen Medien ist, wobei der Dispersion ggf. ein Dispergierhilfsmittel, ein Tensid und/oder ein Verdicker zugesetzt sein kann.

19. Zusammensetzung nach Anspruch 17 oder 18, **dadurch gekennzeichnet, dass** die Zusammensetzung ein Beschichtungs-, Kleb- oder Dichtstoff, ein Polymer, ein Entschäumer, Netz- und/oder Verlaufshilfsmittel, ein Kosmetikum, ein Pflegemittel, ein Medizinprodukt, ein Pharmazeutikum, ein Waschmittel, ein Putz- und/oder Reinigungsmittel, ein Hydrophobierungsmittel, ein Gleitmittel oder ein Trennmittel ist.

## Claims

1. Process for preparing silicone (meth)acrylate particles, comprising the steps of
a) obtaining an emulsion composed of water and an organic phase, said organic phase comprising organopolysiloxanes which have been modified terminally and/or laterally with (meth)acrylate groups and are of the general formula (I) or mixtures thereof 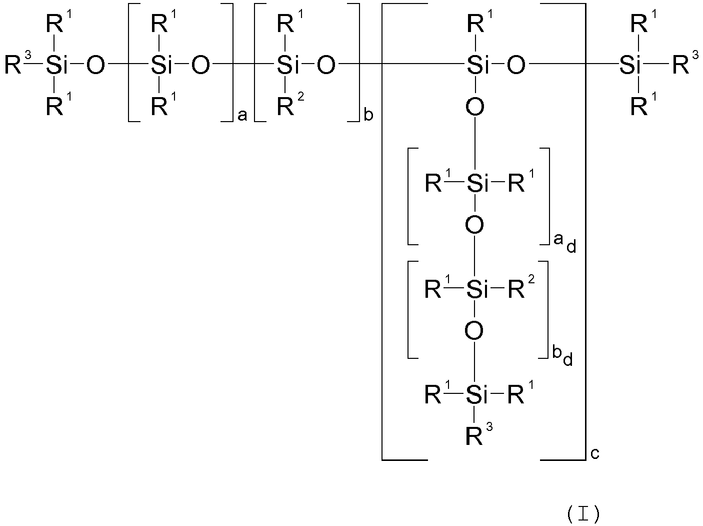 where
R¹ = identical or different radicals selected from linear or branched, saturated, monounsaturated or polyunsaturated, linear, cyclic or branched alkyl, alkoxy, polyalkoxy, hydroxyalkyl, hydroxyalkoxy, alkenyl, aryl, aryloxy, hydroxyaryl, hydroxyaryloxy, alkaryl, alkaryloxy, hydroxyalkaryl, hydroxyalkaryloxy, aralkyl, aralkoxy, hydroxyaralkyl or hydroxyaralkoxy radicals which optionally contain one or more ether or ester bridges and have 1 to 20 carbon atoms,
R² = identical or different divalent, optionally OH-functionalized hydrocarbon radicals which optionally contain one or more ether or ester bridges, are bonded to the silicon atom via an Si-C linkage or an Si-O-C linkage, have 1 to 20 carbon atoms and to which are bonded, via ester bonds, 1 to 5 acrylic acid and/or methacrylic acid units and optionally monocarboxylic acid units having 2 to 10 carbon atoms, which are free of double bonds capable of polymerization,
R³ = identical or different R¹ or R² radicals,
a = 0 to 1000,
b = 0 to 200,
c = 0 to 200,
a_{d} = 0 to 1000,
b_{d} = 0 to 200,
where
the index d, when c > 0, is an integer > 0,
with the proviso that when b and c = 0, R³ must not be selected from the same group as R¹, with addition of at least one emulsifier and optionally of one or more coemulsifiers,
where
the organic phase forms the inner phase of the emulsion, and
b) polymerizing the inner phase to completion in the presence of a free-radical initiator which is added to the outer, aqueous phase in a concentration of 0.1 to 40% by weight based on the inner phase.

2. Process according to Claim 1, **characterized in that** in step a) an emulsion stabilized in the solid state is obtained and the emulsifiers used are particulate emulsifiers which are microscale or nanoscale in at least one dimension and are selected from the group of the metal oxides, mixed oxides, nitrides, hydroxides, carbonates, silicates, silicone resins, silicones and/or organic polymers which are at least partly hydrophobized with at least one compound from the group of the silanes, siloxanes, quaternary ammonium compounds, cationic polymers and fatty acids or anions thereof.

3. Process according to Claim 1 or 2, **characterized in that** more than 70% of the R¹ radicals in formula (I) are methyl groups.

4. Process according to at least one of Claims 1 to 3, **characterized in that** R² in the general formula(I) is selected from the group of the 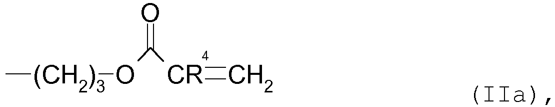 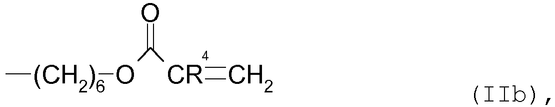 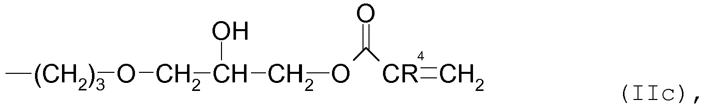 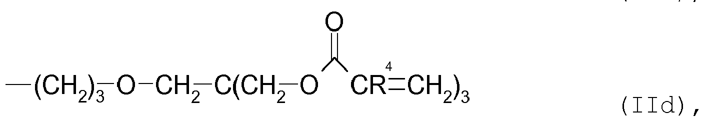 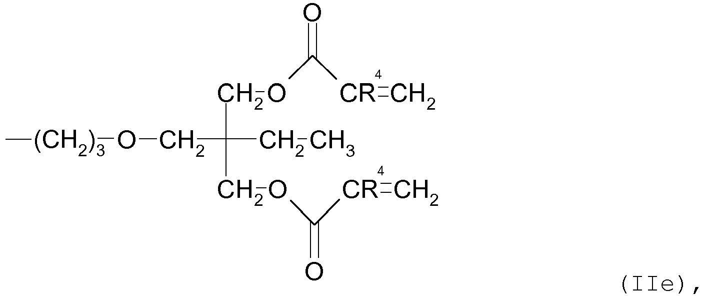 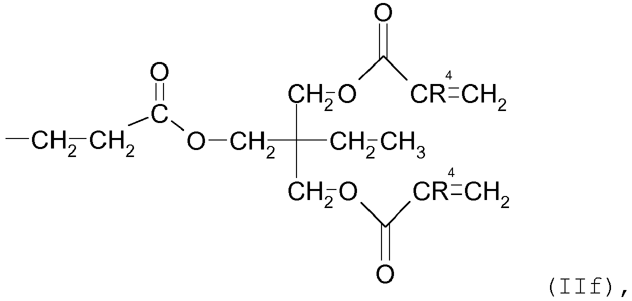 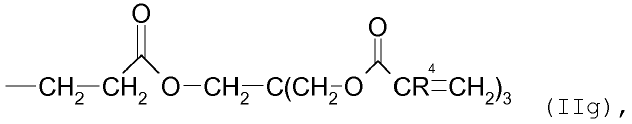 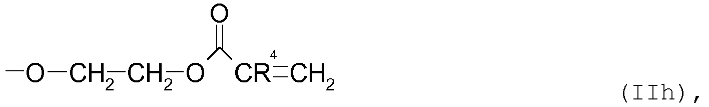 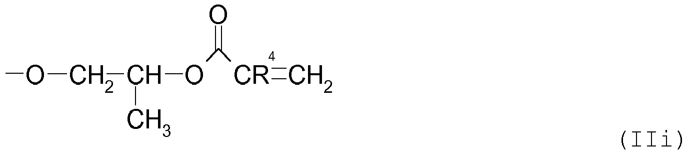 and/or 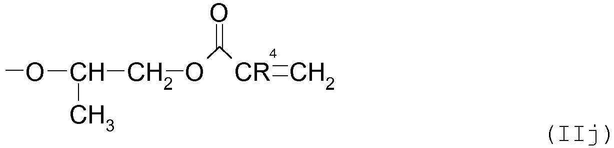 radicals and
R⁴ is hydrogen or a methyl group.

5. Process according to at least one of Claims 1 to 4, **characterized in that** silicone (meth)acrylates of the formula (I) in which a assumes a value of 0 to 500 are used.

6. Process according to at least one of Claims 1 to 5, **characterized in that** the silicone (meth)acrylates of the formula (I) in which b assumes a value of 0 to 100 are used.

7. Process according to at least one of Claims 1 to 6, **characterized in that** the silicone (meth)acrylates of the formula (I) in which c assumes a value of 0 to 100 are used.

8. Process according to at least one of Claims 1 to 7, **characterized in that** further comonomers are added to the organic phase before step b).

9. Process according to at least one of Claims 1 to 8, **characterized in that** further components are added to the organic phase in step a).

10. Process according to at least one of Claims 1 to 9, **characterized in that** the organic phase comprises substances which can be released from the particles.

11. Process according to at least one of Claims 1 to 10, **characterized in that** the emulsion is prepared in step a) by passing the mixture of organic and aqueous phase through and dispersing the mixture in at least one interaction chamber with a capillary thickness of 50 to 500 µm in a pressure range of 50 to 1000 bar and decompressing the mixture into an outlet reservoir.

12. Silicone (meth)acrylate particles obtainable by a process according to any one of Claims 1 to 11.

13. Silicone (meth)acrylate particles according to Claim 12, **characterized in that** the particles possess a core-shell structure, said shell being formed by particulate emulsifiers and surrounding an inner core composed of silicone (meth)acrylate.

14. Silicone (meth)acrylate particles according to Claim 13, **characterized in that** the shell is formed from inorganic particles or is a polymer shell whose surface has been modified.

15. Silicone (meth)acrylate particles according to at least one of Claims 12 to 14, **characterized in that** the particles include substances which are released from the particles.

16. Use of the silicone (meth)acrylate particles according to at least one of Claims 12 to 15 or prepared according to at least one of Claims 1 to 11 alone or in a mixture with further particles, pigments and/or further customary additives in the form of powders or dispersions in coating, adhesive or sealant materials, in polymers, in defoamers, in wetting and levelling aids, in cosmetic or pharmaceutical formulations and care products, in cleaning and detergent compositions or in applications for modifying the interface properties of solid and liquid substrates.

17. Compositions comprising silicone (meth)acrylate particles according to Claims 12 to 15 or prepared according to any one of Claims 1 to 11.

18. Composition according to Claim 17, **characterized in that** it is a dispersion of silicone (meth)acrylate particles in aqueous or organic media, to which dispersion it is optionally possible to add a dispersing aid, a surfactant and/or a thickener.

19. Composition according to Claim 17 or 18, **characterized in that** the composition is a coating, adhesive or sealant material, a polymer, a defoamer, wetting and/or levelling aid, a cosmetic, a care product, a medical product, a pharmaceutical, a washing composition, a cleaning and/or detergent composition, a hydrophobizing agent, a lubricant or a release agent.

## Revendications

1. Procédé de fabrication de particules de (méth)acrylate de silicone comprenant les étapes suivantes :
a) la formation d'une émulsion d'eau et d'une phase organique, la phase organique contenant des organopolysiloxanes modifiés en position terminale et/ou latérale avec des groupes (méth)acrylate selon la formule générale (I) ou leurs mélanges 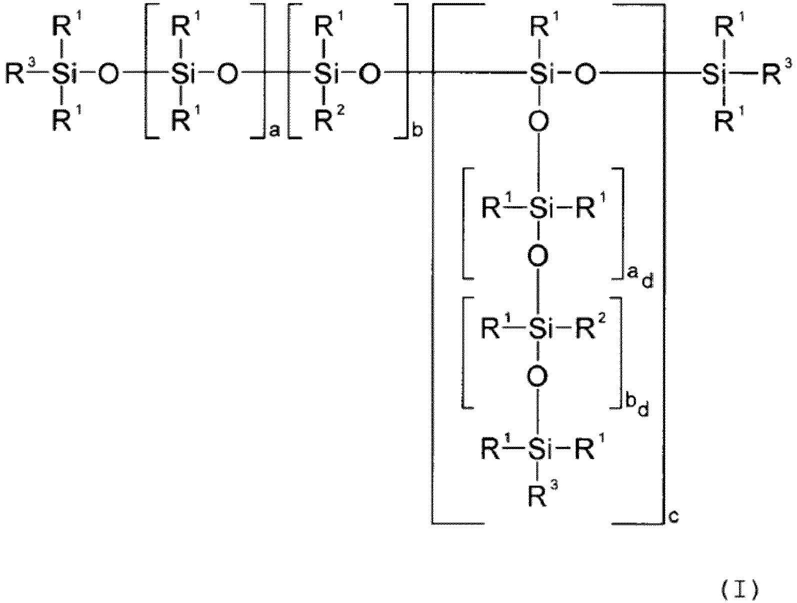 avec
R¹ = des radicaux identiques ou différents, choisis parmi les radicaux alkyle, alcoxy, polyalcoxy, hydroxyalkyle, hydroxyalcoxy, alcényle, aryle, aryloxy, hydroxyaryle, hydroxyaryloxy, alkaryle, alkaryloxy, hydroxyalkaryle, hydroxyalkaryloxy, aralkyle, aralcoxy, hydroxyaralkyle ou hydroxyaralcoxy de 1 à 20 atomes C linéaires, cycliques ou ramifiés, contenant éventuellement un ou plusieurs ponts éther ou ester, linéaires ou ramifiés, saturés, mono- ou polyinsaturés,
R² = des radicaux hydrocarbonés identiques ou différents, éventuellement fonctionnalisés par OH, bivalents, contenant éventuellement un ou plusieurs ponts éther ou ester, reliés à l'atome Si par une liaison Si-C ou une liaison Si-O-C, de 1 à 20 atomes C, auxquels 1 à 5 unités acide acrylique et/ou acide méthacrylique et éventuellement unités acide monocarboxylique de 2 à 10 atomes C, qui sont exemptes de doubles liaisons aptes à la polymérisation, sont reliées par des liaisons ester, R³ = des radicaux R¹ ou R² identiques ou différents,
a = 0 à 1 000,
b = 0 à 200,
c = 0 à 200,
a_{d} = 0 à 1 000,
b_{d} = 0 à 200,
l'indice d pour c > 0 étant un nombre entier > 0,
à condition que lorsque b et c = 0, R³ ne puisse pas être choisi dans le même groupe que R¹,
avec ajout d'au moins un émulsifiant et éventuellement d'un ou de plusieurs co-émulsifiants,
la phase organique formant la phase intérieure de l'émulsion, et
b) la polymérisation de la phase intérieure en présence d'un démarreur radicalaire, qui est ajouté dans la phase aqueuse extérieure en une concentration de 0,1 à 40 % en poids, par rapport à la phase intérieure.

2. Procédé selon la revendication 1, **caractérisé en ce qu'**à l'étape a), une émulsion stabilisée par des solides est formée et, en tant qu'émulsifiants, des émulsifiants particulaires, micro- ou nanométriques dans au moins une dimension, qui sont choisis dans le groupe constitué par les oxydes de métaux, les oxydes mixtes, les nitrures, les hydroxydes, les carbonates, les silicates, les résines de silicone, les silicones et/ou les polymères organiques, qui sont au moins (partiellement) hydrophobés avec au moins un composé du groupe constitué par les silanes, les siloxanes, les composés d'ammonium quaternaires, les polymères cationiques et les acides gras ou leurs ions, sont utilisés.

3. Procédé selon la revendication 1 ou 2, **caractérisé en ce que** plus de 70 % des radicaux R¹ dans la formule (I) sont des groupes méthyle.

4. Procédé selon au moins l'une quelconque des revendications 1 à 3, **caractérisé en ce que** R² dans la formule générale (I) est choisi dans le groupe constitué par les radicaux 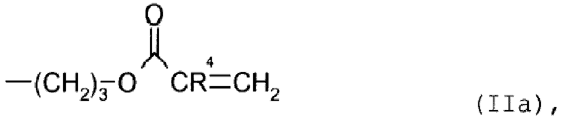 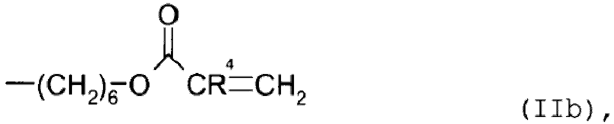 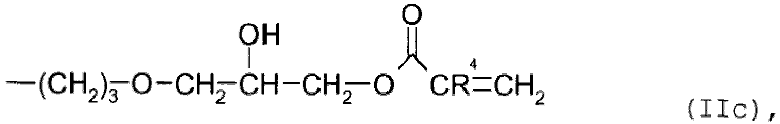 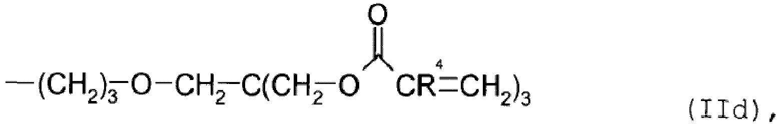 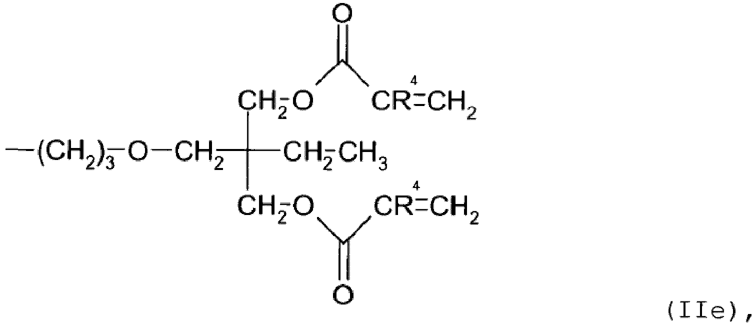 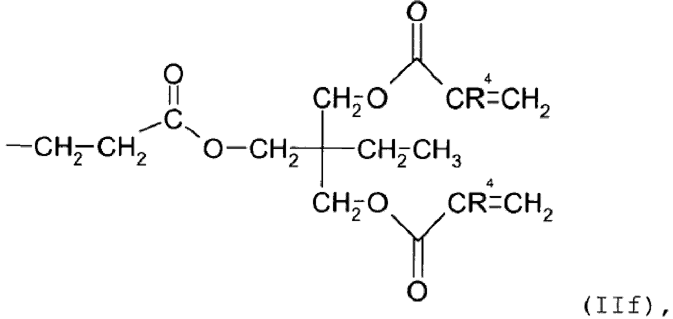 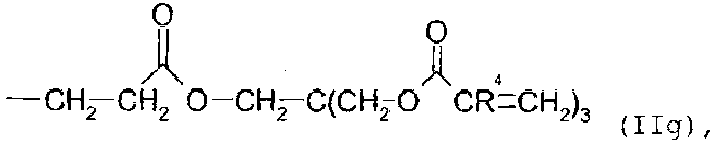 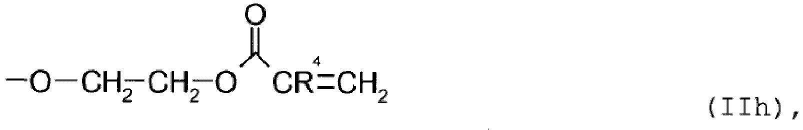 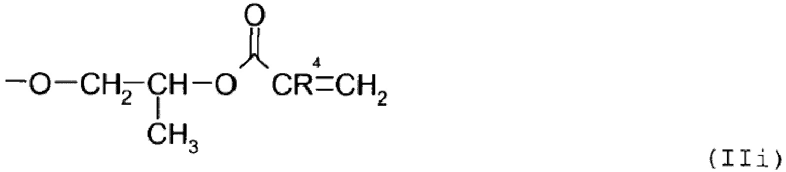 et/ou 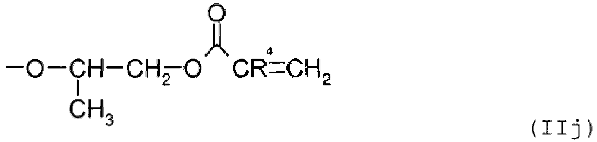 et
R⁴ est l'hydrogène ou un groupe méthyle.

5. Procédé selon au moins l'une quelconque des revendications 1 à 4, **caractérisé en ce que** des (méth)acrylates de silicone de formule (I) sont utilisés, dans lesquels a prend une valeur de 0 à 500.

6. Procédé selon au moins l'une quelconque des revendications 1 à 5, **caractérisé en ce que** des (méth)acrylates de silicone de formule (I) sont utilisés, dans lesquels b prend une valeur de 0 à 100.

7. Procédé selon au moins l'une quelconque des revendications 1 à 6, **caractérisé en ce que** des (méth)acrylates de silicone de formule (I) sont utilisés, dans lesquels c prend une valeur de 0 à 100.

8. Procédé selon au moins l'une quelconque des revendications 1 à 7, **caractérisé en ce que** des comonomères supplémentaires sont ajoutés à la phase organique avant l'étape b).

9. Procédé selon au moins l'une quelconque des revendications 1 à 8, **caractérisé en ce que** des composants supplémentaires sont ajoutés à la phase organique à l'étape a).

10. Procédé selon au moins l'une quelconque des revendications 1 à 9, **caractérisé en ce que** la phase organique contient des substances qui peuvent être libérées par les particules.

11. Procédé selon au moins l'une quelconque des revendications 1 à 10, **caractérisé en ce que** l'émulsion est fabriquée à l'étape a) par passage du mélange d'une phase organique et d'une phase aqueuse au travers de et dispersion du mélange dans au moins une chambre d'interaction ayant une épaisseur capillaire de 50 à 500 µm dans une plage de pression allant de 50 à 1 000 bar et détente du mélange dans un réservoir de sortie.

12. Particules de (méth)acrylate de silicone, pouvant être obtenues par un procédé selon l'une quelconque des revendications 1 à 11.

13. Particules de (méth)acrylate de silicone selon la revendication 12, **caractérisées en ce que** les particules présentent une structure noyau-enveloppe, l'enveloppe étant formée par des émulsifiants particulaires, et entourant un noyau intérieur en (méth)acrylate de silicone.

14. Particules de (méth)acrylate de silicone selon la revendication 13, **caractérisées en ce que** l'enveloppe est formée par des particules inorganiques ou est une enveloppe polymère dont la surface est modifiée.

15. Particules de (méth)acrylate de silicone selon au moins l'une quelconque des revendications 12 à 14, **caractérisées en ce que** les particules contiennent des substances qui sont libérées par les particules.

16. Utilisation des particules de (méth)acrylate de silicone selon au moins l'une quelconque des revendications 12 à 15 ou fabriquées selon au moins l'une quelconque des revendications 1 à 11 seules ou en mélange avec d'autres particules, pigments et/ou d'autres additifs usuels sous la forme de poudres ou de dispersions dans des agents de revêtement, des agents adhésifs ou des agents d'étanchéité, dans des polymères, dans des antimousses, dans des adjuvants de mouillage et d'étalement, dans des préparations cosmétiques ou pharmaceutiques, ainsi que des agents de soin, dans des agents brillants et détergents, ou dans des applications pour la modification des propriétés d'interface de substrats solides et liquides.

17. Compositions contenant des particules de (méth)acrylate de silicone selon les revendications 12 à 15 ou fabriquées selon l'une quelconque des revendications 1 à 11.

18. Composition selon la revendication 17, **caractérisée en ce que** la composition est une dispersion de particules de (méth)acrylate de silicone dans des milieux aqueux ou organiques, un adjuvant de dispersion, un tensioactif et/ou un épaississant pouvant éventuellement être ajoutés à la dispersion.

19. Composition selon la revendication 17 ou 18, **caractérisée en ce que** la composition est un agent de revêtement, un agent adhésif ou un agent d'étanchéité, un polymère, un antimousse, un adjuvant de mouillage et/ou d'étalement, un produit cosmétique, un agent de soin, un produit médical, un produit pharmaceutique, un agent de lavage, un agent brillant et/ou détergent, un agent hydrophobant, un agent lubrifiant ou un agent de démoulage.
